# EUROPEAN PATENT SPECIFICATION

(11) **EP 1 322 641 B1**
(45) Date of publication and mention of the grant of the patent: **25.07.2012**
(21) Application number: 01967621.2
(22) Date of filing: 27.09.2001
(51) Int. Cl.: C07D 409/12, A61K 31/4535, A61P 9/10, A61P 19/02, A61P 25/00, A61P 35/00

(54) **PHARMACEUTICALLY ACTIVE HYDROPHILIC SULFONAMIDE DERIVATIVES AS INHIBITORS OF PROTEIN JUNKINASES**
PHARMAZEUTISCH WIRKSAME HYDROPHILE SULFONAMIDDERIVATE ZUR VERWENDUNG ALS PROTEINJUNKINASEHEMMER
DERIVES SULFONAMIDIQUES HYDROPHILES, ACTIFS DU POINT DE VUE PHARMACEUTIQUE, UTILISES COMME INHIBITEURS DES PROTEINE JUN-KINASES

(30) Priority: 27.09.2000 EP 00810886
(43) Date of publication of application: 02.07.2003
(73) Proprietor: Merck Serono SA, 1267 Coinsins, Vaud (CH)
(72) Inventor: HALAZY, Serge, F-74100 Vetraz-Monthoux (FR); CHURCH, Dennis, CH-1291 Commugny (CH); CAMPS, Montserrat, CH-1290 Versoix (CH); GOTTELAND, Jean-Pierre, F-74160 Beaumont (FR); RUECKLE, Thomas, CH-1228 Plan-les-Ouates (CH); BIAMONTE, Marco, US-92124 CA San Diego (US); ARKINSTALL, Stephen, Belmont, MA 02478 (US)
(74) Representative: Merck Serono SA - Geneva Intellectual Property
(86) International application number: PCT/IB2001/001771
(87) International publication number: WO 2002/028856

(56) References cited:
- EP-A- 0 138 720
- WO-A-00/02851
- WO-A-99/65451

## Description

### Field of the invention

The present invention is related to substantially hydrophilic sulfonamide derivatives or sulfonamide derivatives having a substantially hydrophilic moiety. Said sulfonamide derivatives are notably for use as pharmaceutically active compounds. Also, the present invention is related to pharmaceutical formulations containing such sulfonamide derivatives. In particular, the present invention is related to sulfonamide derivatives that are useful in the treatment and/or prevention of disorders of the immune and the neuronal system. Specifically, the sulfonamide derivatives of the present invention display a substantial modulatory, notably an inhibitory activity of the JNK (Jun-Kinase) function or pathways respectively.

### Background of the invention

Apoptosis denotes the complex contortions of the membrane and organelles of a cell as it undergoes the process of programmed cell death. During said process, the cell activates an intrinsic suicide program and systematically destroys itself. The following series of events can be observed:
- The cell surface begins to bleb and expresses pro-phagocytic signals. The whole apoptotic cell then fragments into membrane-bound vesicles that are rapidly and neatly disposed of by phagocytosis, so that there is minimal damage to the surrounding tissue.
- The cell then separates from its neighbors.

The nucleus also goes through a characteristic pattern of morphological changes as it commits genetic suicide, the chromatin condenses and is specifically cleaved to fragments of DNA.

Neuronal cell death plays an important role in ensuring that the nervous system develops normally. It appears that the death of developing neurons depends on the size of the target that they innervate: cells with fewer synaptic partners are more likely to die than those that have formed multiple synapses. This may reflect a process, which balances the relative number of pre- to postsynaptic neurons in the developing nervous system. Although neuronal cell death was assumed to be apoptotic, it was only recently that neurons in developing rodent brain were conclusively shown to undergo apoptosis as classified by morphology and DNA fragmentation. As cell death during development is clearly not a pathological process, it makes sense that cells actually cease to exist.

Neuronal death occurs via either apoptotic or necrotic processes following traumatic nerve injury or during neurodegenerative diseases. Multiple components are emerging as key players having a role in driving neuronal programmed cell death. Amongst the components leading to neuronal apoptosis are members of the SAPK/JNK being a sub-family of MAP Kinases (MAPKs).

Mammalian cells respond to some extracellular stimuli by activating signaling cascades which are mediated by various mitogen-activated protein kinases (MAPKs). Despite the differences in their response to upstream stimuli, the MAP kinase cascades are organized in a similar fashion, consisting of MAP kinase kinase kinases (MAPKKK or MEKK), MAP kinase kinases (MAPKK or MKK) and MAP kinases (MAPK). MAP kinases are a broad family of kinases which includes c-Jun N-Terminal kinases (JNKs), also known as "stress-activated protein kinases" (SAPKs), as well as extracellular signal regulated kinases (ERKs) and p38 MAP kinases. Each of these three MAP kinases subfamilies is involved in at least three different but parallel pathways conveying the information triggered by external stimuli. The JNK signaling pathway is activated by exposure of cells to environmental stress -such as chemical toxins, radiation, hypoxia and osmotic shock- as well as by treatment of cells with growth factors or pro-inflammatory cytokines -such as tumour necrosis factor alpha (TNF-α) or interleukin-1 beta (IL-1β).

Two MAP kinase kinases (known as MKKs or MAPKKs), i.e. MKK4 (known also as JNKK1) and MKK7 (known also as JNKK2), activate JNK by a dual phosphorylation of specific threonine and tyrosine residues located within a Thr-Pro-Tyr motif on the activation loop on the enzyme, in response to cytokines and stress signals. Even further upstream in the signaling cascade, MKK4 is known to be activated itself also by a MAP kinase kinase kinase, MEKK1 through phosphorylation at serine and threonine residues. Once activated, JNK binds to the N-terminal region of transcription factor targets and phosphorylates the transcriptional activation domains resulting in the up-regulation of expression of various gene products, which can lead to apoptosis, inflammatory responses or oncogenic processes **(1-5)**.

MAPKs (mitogen-activated protein kinases) are serine/threonine kinases that are activated by dual phosphorylation on threonine and tyrosine residues. In mammalian cells, there are at least three separate but parallel pathways that convey information generated by extra-cellular stimuli to the MAPKs. Said pathways consist of kinase cascades leading to activation of the ERKs (extracellular regulated kinases), the JNKs (c-Jun N-terminal kinases), and the p38/CSBP kinases. While both the JNK and p38 pathways are involved in relaying stress-type extramolecular signals, the ERK pathway is primarily responsible for transducing mitogenic/differentiation signals to the cell nucleus.

SAPK cascades represent a sub-family of the mitogen-activating protein kinase family, that are activated by different external stimuli including DNA damage following UV irradiation, TNF-α, IL-1β, ceramide, cellular stress, and reactive oxygen species and have distinct substrate specificities. Signal transduction via MKK4/JNK of MKK3/p38 results in the phosphorylation of inducible transcription factors, c-Jun and ATF2, which then act as either homodimers or heterodimers to initiate transcription of down-stream effectors.

c-Jun is a protein that is forming homodimers and heterodimers (with e.g. c-Fos) to produce the transactivating complex AP-which is required for the activation of many genes (e.g. matrix metalloproteinases) involved in the inflammatory response. The JNKs were discovered when it was found that several different stimuli such as UV light and TNF-α stimulated phosphorylation of c-Jun on specific serine residues in the N-terminus of the protein.

Three distinct JNK enzymes have been identified as products of the genes JNK1, JNK2 and JNK3 and ten different isoforms of JNK have been identified **(3, 6, 7)**. JNK1 and -2 are ubiquitously expressed in human tissues, whereas JNK3 is selectively expressed in the brain, heart and testes **(7, 8, 9, 10)**. Each isoform binds to the substrates with different affinities, suggesting, *in vivo,* a substrate specific regulation of the signaling pathways by the different JNK isoforms.

In a recent publication of Xie X et al, (Structure 1998, 6 (8); 983-991) it has been suggested that activation of stress-activated signal transduction pathways are required for neuronal apoptosis induced by NGF withdrawal in rat PC-12 and superior cervical ganglia (SCG) sympathetic neuronal cells. Inhibition of specific kinases, namely MAP kinase kinase 3 (MKK3) and MAP kinase kinase 4 (MKK4), or c-Jun (part of the MKK-4 cascade) may be sufficient to block apoptosis (see also Kumagae Y et al, in Brain Res Mol Brain Res, 1999, 67(1), 10-17 and Yang DD et al in Nature, 1997, 389 (6653); 865-870). Within a few hours of NGF deprivation in SCG neurones, c-Jun becomes highly phosphorylated and protein levels increase. Similarly in rat PC-12 cells deprived of NGF, JNK and p38 undergo sustained activation while ERKs are inhibited. Consistent with this JNK3 KO mice are resistant to excitotoxicity induced apoptosis in the hippocampus and more importantly they display greatly reduced epileptic like seizures in response to excitotoxicity as compared to normal animals (Nature 1997, 389, 865-870). More recently, it has been reported that the JNK signalling pathway is implicated in cell proliferation and could play an important role in autoimmune diseases (Immunity, 1998, 9, 575-585; Current Biology, 1999, 3, 116-125) which are mediated by T-cell activation and proliferation.

Naive (precursor) CD4⁺helper T (Th) cells recognise specific MHC-peptide complexes on antigen-presenting cells (APC) via the T-cell receptor (TCR) complex. In addition to the TCT-mediated signal, a co-stimulatory signal is provided at least partially by the ligation of CD28 expressed on T-cells with B7 proteins on APC. The combination of these two signals induces T-cell clonal expression.

After 4-5 days of proliferation, precursor of CD4⁺ T cells differentiate into armed effector Th cells that mediate the functions of the immune system. During the differentiation process, substantial reprogramming of gene expression occurs.

Two subsets of effector Th cells have been defined on the basis of their distinct cytokine secretion pattern and their immuno-modulatory effects: Th1 cells produce IFNγ and LT (TNF-β), which are-required for cell-mediated inflammatory reactions; Th2 cells secrete IL-4, IL-5, IL-6, IL-10 and IL-13, which mediate B cell activation and differentiation. These cells play a central role in the immune response. The JNK MAP Kinase pathway is induced in Th1 but not in Th2 effector cells upon antigen stimulation. Furthermore, the differentiation of precursor CD4⁺T cells into effector Th1 but not Th2 cells is impaired in JNK2-deficient mice. Therefore, in recent years it has been realised that the JNK kinase pathway plays an important role in the balance of Th1 and Th2 immune response through JNK2.

Some transcription factors known to be JNK substrates are the Jun proteins (c-jun, JunB and Jun D), the related transcription factors ATF2 and ATFa, Ets transcription factors such as Elk-1 and Sap-1, the tumor suppressor p53 and a cell death domain protein (DENN).

Activation of the JNK pathway has been documented in a number of disease processes, thus providing a rationale for targeting this pathway for drug discovery. In addition, molecular genetic approaches have validated the pathogenic role of this pathway in several diseases.

For example, auto-immune and inflammatory diseases derive from the inappropriate activation of the immune system. Activated immune cells express many genes encoding inflammatory molecules, including cytokines, growth factors, cell surface receptors, cell adhesion molecules and degradative enzymes. Many of these genes are known to be regulated by the JNK pathway, through the activation of the transcription factors c-Jun and ATF-2.

The inhibition of JNK activation in bacterial lipopolysaccharide-stimulated macrophages, effectively modulates the production of the key pro-inflammatory cytokine, TNFα **(11)**.

The inhibition of JNK activation decreases the transcription factor activation responsible of the inducible expression of matrix metalloproteinases (MMPs) **(12)**, which are known to be responsible of the promotion of cartilage and bone erosion in rheumatoid arthritis and of generalized tissue destruction in other auto-immune diseases.

The JNK cascade is also activated in T cells by antigen stimulation and CD28 receptor co-stimulation **(13)** and regulates the production of the IL-2 promoter **(14).** Inappropriate activation of T lymphocytes initiates and perpetuates many auto-immune diseases, including asthma, inflammatory bowel syndrome and multiple sclerosis.

In neurons vulnerable to damage from Alzheimer's disease and in CA1 neurons of patients with acute hypoxia **(15),** JNK3 protein is highly expressed. The JNK3 gene was also found to be expressed in the damaged regions of the brains of Alzheimer's patients **(16).** In addition, neurons from JNK3 KO mice were found to become resistant to kainic acid induced neuronal apoptosis compared to neurons from wild-type mice **(8).**

Based on these findings, the JNK signaling pathway and especially that of JNK2 and JNK3, is thought to be implicated in apoptosis-driven neurodegenerative diseases such as Alzheimer's disease, Parkinson's disease, epilepsy and seizures, Huntington's disease, traumatic brain injuries as well as ischemic and hemorrhaging strokes.

Cardiovascular diseases, such as atherosclerosis and restenosis result from defective regulation of growth of the blood vessel wall. The JNK pathway is activated by atherogenic stimuli and regulates local cytokine and growth factor production in vascular cells **(17,18)** inducing pro-atherosclerotic gene **(19).**

Ischemia alone or coupled with reperfusion in the heart, liver, kidney or brain results in cell death and scar formation, which can ultimately lead to congestive heart failure, hepatic disorders, renal failure or cerebral dysfunction. The JNK pathway is activated by ischemia and reperfusion in the heart **(20),** leading to the activation of JNK-responsive genes and leukcocyte-mediated tissue damage. JNK activation is also observed in kidney **(21)** or liver **(22)** following ischemia and reperfusion. The down-regulation of JNKs has been proven to improve renal function and long-term outcome during nephritic and ischemic renal failure **(23).**

Cancer is characterized by uncontrolled growth, proliferation and migration of cells. In early lung cancer, expression of c-jun is altered and may mediate growth factor signaling in non-small cell lung cancer **(24)**. In addition to regulating *c*-jun production and activity, JNK activation can regulate phosphorylation of p53, and thus can modulate cell cycle progression **(25).** Moreover, the role of JNK activation in HTLV-1 (human T cell leukemia virus type 1) mediated tumorgenesis **(26)** suggests the potential use of JNK inhibitors in cancer treatment **(27).** Selective inhibition of JNK activation by a naturally occuring JNK inhibitory protein, called JNK-interacting-protein-1 (JIP1), blocks cellular transformation **(28).** Thus, JNK inhibitors may block transformation and tumor cell growth.

With the objective of inhibiting the JNK kinase pathway, WO/9849188 teaches the use of a human polypeptide, i.e. INK-interacting protein 1 (JIP-1), which is a biological product and which has also been assayed for overcoming apoptosis related disorders.

Although such human polypeptides have been confirmed to have an inhibitory effect onto the JNK kinase pathway, a whole variety of drawbacks are associated with their use :
- Active bio-peptides or bio-proteins are only obtained by means of rather comprehensive and expensive bio-synthesis which consequently frequently renders the resulting products fairly cost-intensive.
- The peptides are known to display poor membrane penetration and may not cross the blood brain membrane,
- The principal drawback to the use of peptide inhibitors or antagonists is the problem of low oral bioavailability resulting from intestinal degradation. Hence, they must be administered parenterally and finally,
- peptide inhibitors or antagonists are frequently viewed by the host body as intruding material to be eliminated, thus setting off an auto-immune response.

The high relevance of the JNK pathway in some widely spread diseases stresses the need to develop inhibitors, preferentially selective, of JNKs.

It is therefore an objective of the present invention to provide molecules which are suitable for the treatment of a variety of diseases, in particular of neuronal or the autoimmune system related disorders, cancer, ischemic conditions and cardiovascular diseases. It is notably an objective of the present invention to provide chemical compounds which are able to modulate, preferably to down-regulate or to inhibit the JNK (Jun kinase) pathway so to be useful in method of treating diseases which involve the JNK pathway. Moreover, it is an objective of the present invention to provide methods for preparing said chemical compounds. It is furthermore an objective of the present invention to provide a new category of pharmaceutical formulations for the treatment of diseases, in particular those mediated by the JNK function.

It is finally an objective of the present invention to provide a method for the treatment and/or prevention of diseases that are caused by disorders of the autoimmune and/or the neuronal system.

### Description of the invention

The aforementioned objectives have been met according to the independent claims. Preferred embodiments are set out within the dependent claims which are incorporated herewith.

The following paragraphs provide definitions of the various chemical moieties that make up the compounds according to the invention and are intended to apply uniformly throughout the specification and claims unless an otherwise expressly set out definition provides a broader definition.

"C₁-C₆-alkyl" refers to monovalent alkyl groups having 1 to 6 carbon atoms. This term is exemplified by groups such as methyl, ethyl, n-propyl, isopropyl, n-butyl, isobutyl, tert-butyl, n-hexyl.

"Aryl" refers to phenyl.

"Alkenyl" refers to alkenyl groups preferably having from 2 to 6 carbon atoms and having at least 1 or 2 sites of alkenyl unsaturation. Preferable alkenyl groups include ethenyl (-CH=CH₂), n-2-propenyl (allyl, -CH₂CH=CH₂)."Alkoxy" refers to the group - O-R where R is "C₁-C₆-alkyl". Preferred alkoxy groups include by way of example, methoxy, ethoxy.

"Halogen," refers to fluoro, chloro, promo and iodo atoms.

"Sulfonyl" refers to group "-SO₂-R" wherein R is selected from H, "aryl", "C₁-C₆-alkyl", "C₁-C₆-alkyl" substituted with halogens e.g. an -SO₂-CF₃ group, "C₁-C₆-alkyl aryle

"Pharmaceutically acceptable salts" refers to salts of the below-identified compounds of formula I that retain the desired biological activity. Examples of such salts include, but are not restricted to acid addition salts formed with inorganic acids (e.g. hydrochloric acid, hydrobromic acid, sulfuric acid, phosphoric acid, nitric acid,), and salts formed with organic acids such as acetic acid, oxalic acid, tartaric acid, succinic acid, malic acid, fumaric acid, maleic acid, ascorbic acid, benzoic acid, tannic acid, pamoic acid, alginic acid, polyglutamic acid, naphthalene sulfonic acid, naphthalene disulfonic acid, and polygalacturonic acid. Said compounds can also be administered as pharmaceutically acceptable quaternary salts known by a person skilled in the art, which specifically include the quarternary ammonium salt of the formula -NR,R',R"⁺ Z⁻, wherein R, R', R" is independently hydrogen, alkyl, or benzyl, and Z is a counterion, including chloride, bromide, iodide, -O-alkyl, toluenesulfonate, methylsulfonate, sulfonate, phosphate, or carboxylate (such as benzoate, succinate, acetate, glycolate, maleate, malate, fumarate, citrate, tartrate, ascorbate, cinnamoate, mandeloate, and diphenylacetate). Sample based-addition salts include those derived from sodium, potassium, ammonium, and quaternary ammonium hydroxide, such as for example tetramethylammonium hydroxide.

"Hydrophilic group" refers to functional groups which have a pronounced attraction to hydrophilic or polar groups, substituents or compounds or fatty compounds or moieties. "Enantiomeric excess" (ee) refers to the products that are obtained by an essentially enantiomeric synthesis or a synthesis comprising an enantioselective step, whereby a surplus of one enantiomer in the order of at least about 52% ee is yielded. In the absence of an enantiomeric synthesis, racemic products are usually obtained that do however also have the inventive set out activity as of JunKinases inhibitors.

One aspect of the present invention consists in sulfonamide derivatives according to formula I :

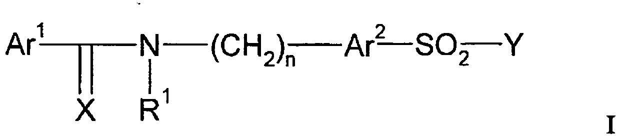

The compounds of formula I according to the present invention being suitable pharmaceutical agents are those wherein
Ar¹ is a phenyl optionally substituted by -OR wherein R is C₁-C₆-alkyl;
Ar² is a thienyl group carrying at least one hydrophilic substituent wherein the hydrophilic substituent is -COOR³, -CONR³R³', OH, C₁-C₄alkyl substituted with OH or an amino group, a hydrazido carbonyl group, a sulfate, a sulfonate, an amine or an ammonium salt;
X is O or S, preferably O.
R¹ is hydrogen or a C₁-C₆-alkyl group.
n is an integer from 1-3 and most preferred 1.

Y is a piperidine moiety according to the below formula

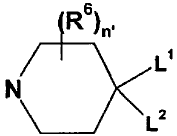

In said piperidine group, L¹ and L² are independently selected from the group consisting of H, -NR^{3'}R³, -NR^{3'}C(O)R³, -NR^{3'}C(O)NR^{3'}R³, -(SO)R³, -(SO₂)R³, -NSO₂R³, - SO₂NR^{3'}R³_{.}

Thereby, R³ and R^{3'} are substituents independently selected from the group consisting of H, C₁-C₆-alkyl, C₂-C₆-alkenyl, aryl being phenyl, aryl-C₁-C₆-alkyl being phenyl-C₁₋C₆-alkyl, R⁶ is selected from the group consisting of hydrogen, C₁-C₆-alkyl, C₁-C₆-alkoxy, OH, halogen, nitro, cyano, sulfonyl, oxo (=O), and n' is an integer from 0 to 4, preferably 1 or 2.

All of the above mentioned aryl groups could optionally be substituted by at least one of the groups selected from halogen, hydroxy, nitro, sulfonyl.
The present invention also includes the geometrical isomers, the optical active forms, enantiomers, diastereomers of compounds according to formula I, as well as their racemates and also pharmaceutically acceptable salts of the sulfonamide derivatives of formula I.

Ar¹ in formula I are those that are independently selected from the group consisting of phenyl, optionally substituted by C₁-C₆-alkoxy, Ar² is a thienyl group with at least one, preferably one hydrophilic substituent selected from -COOR³, -CONR³R^{3'}, OH, C₁-C₄alkyl substituted with OH or an amino group, a hydrazido carbonyl group, a sulphate, a sulfonate, an amine or an ammonium salt.

Particularly preferred sulfonamides are those wherein Ar¹ is a phenyl group, X is O, R¹ is hydrogen, n is 1, Ar² is a thienyl group with one hydrophilic substituent selected from -COOR³, -CONR³R^{3'}, OH, C₁-C₄alkyl substituted with OH or an amino group, a hydrazido carbonyl group, a sulphate, a sulfonate, an amine or an ammonium salt.

Said aryl groups could optionally be substituted by halogen, hydroxy, nitro, sulfonyl.

Specific examples of compounds of formula I include the following :
5-{[(3-methoxybenzoyl)amino]methyl}-2-[(4-{3-[(trifluoromethyl)sulfonyl]anilino}-piperidin-1-yl)sulfonyl]thiophene-3-carboxylic acid
5-{[(3-methoxybenzoyl)amino]methyl}-2-{[4-(octylamino)piperidin-1-yl]sulfonyl}-thiophene-3-carboxylic acid
N-(2-hydroxyethyl)-5-{[(3-methoxybenzoyl)amino]methyl}-2-[(4-{3-[(trifluoromethyl)sulfonyl]anilino}piperidin-1-yl)sulfonyl]thiophene-3-carboxamide N-({4-(hydrazinocarbonyl)-5-[(4-{3-[(trifluoromethyl)sulfonyl]anilino}piperidin-1-yl)sulfonyl]thien-2-yl}methyl)-3-methoxybenzamide
5-{[(3-methoxybenzoyl)amino]methyl}-2-[(4-{3-[(trifluoromethyl)sulfonyl]anilino}-piperidin-1-yl)sulfonyl]thiophene-3-carboxamide
N-[2-(dimethylamino)ethyl]-5-{[(3-methoxybenzoyl)amino]methyl}-2-[(4-{3-[(trifluoromethyl)sulfonyl]anilino}piperidin-1-yl)sulfonyl]thiophene-3-carboxamide N-({4-(hydroxymethyl)-5-[(4-{3-[(trifluoromethyl)sulfonyl]anilino}piperidin-1-yl)sulfonyl]thien-2-yl}methyl)-3-methoxybenzamide 2-{[4-(hexylamino)-1-piperidinyl]sulfonyl}-5-{[(3-methoxybenzoyl)amino]methyl}-3-thiophenecarboxylic acid
5-{[(3-methoxybenzoyl)amino]methyl}-2-[(4-{[4-(trifluoromethyl)benzyl]amino}-1-piperidinyl)sulfonyl]-3-thiophenecarboxylic acid

The compounds of formula I are suitable for use in treating disorders of the immune system and neuronal system of mammals, notably of human beings. Such neuronal system disorders include for example neurodegenerative diseases e.g. Alzheimer's disease, Huntington's disease, Parkinson's disease, retinal diseases, spinal cord injury, multiple sclerosis, head trauma, epilepsy and seizures, ischemic and hemorragic brain strokes. Immune system disorders include for example asthma, transplant rejection, inflammatory processes such as inflammatory bowel disease (IBD), cartilage and bone erosion disorders, rheumatoid arthritis, septic shock.

The compounds according to formula I are also suitable for use in treating cancers, such as breast, colorectal, pancreatic, prostate, testicular, ovarian, lung, liver and kidney cancers.

In another embodiment, the compounds according to formula I may be used for treating cardiovascular diseases including atherosclerosis, restenosis, stroke, ischemia, e.g. cerebral ischemia, myocordial infarction.

In another embodiment, the compounds according to formula I may be used for treating various ischemic conditions including heart and kidney failures, hepatic disorders and brain reperfusion injuries.

Preferably, the compounds according to formula I, alone or in the form of a pharmaceutical composition, are useful for the modulation of the JNK pathway, more specifically for treatment or prevention of disorders associated with expression or activity of JNK, notably of JNK2 and -3. Said modulation usually preferably involves the inhibition of the JNK pathways, notably of the JNK2 and/or -3. Such an abnormal expression or activity of JNK may be triggered by numerous stimuli (e.g. stress, septic shock, oxidative stress, cytokines) and may cause a cascade of processes, leading to, for example, uncontrolled apoptosis, inflammatory responses or oncogenic processes. These phenomena are frequently involved in various disorders including the above enumerated disorders and disease states. Hence, the compounds according to the invention may be used for the treatment of disorders by modulating the JNK function or signaling pathways. The modulation of the JNK function or pathways may involve its activation, but preferably it involves the down-regulation up to inhibition of the JNK pathways, notably of JNK1 and/or -2 and/or JNK3. The compounds of the invention may be employed alone or in combination with further pharmaceutical agents, e.g. with a further JNK modulator. Still a further object of the present invention is a process for preparing the novel sulfonamide derivatives according to formula I which have been set out above. The sulfonamide derivatives of this invention can be prepared from readily available starting materials using the following general methods and procedures. It will be appreciated that where typical or preferred experimental conditions (i.e., reaction temperatures, time, moles of reagents, solvents.) are given, other experimental conditions can also be used unless otherwise stated. Optimum reaction conditions may vary with the particular reactants or solvent used, but such conditions can be determined by one skilled in the art by routine optimisation procedures.

In a preferred method of synthesis, the sulfonamide derivatives of the invention are prepared by first coupling an amine of formula II: 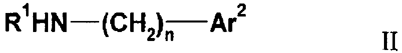 where Ar² and R¹ are as defined above, with an acyl chloride of formula III: 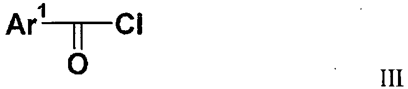 where Ar¹ is as defined above, to provide an amide of formula IV:

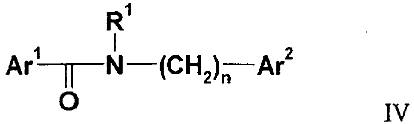

Amines of formula II are either known compounds or can be prepared from known compounds by conventional procedures. Preferred amines as starting materials include thien-2-yl-methylamine.

The acyl chlorides of formula III are also commercially available or previously described compounds. Preferred acyl chlorides include 3-methoxy-benzoyl chloride. If not known, the acid halide can be prepared by reacting the corresponding carboxylic acid with an inorganic acid halide, such as thionyl chloride, phosphorus trichloride or oxalyl chloride under conventional conditions.

Generally, this reaction is performed upon using about 1 to 5 molar equivalents of the inorganic acid halide or oxalyl chloride, either in pure form or in an inert solvent, such as carbon tetrachloride, at temperature in the range of about 0°C to about 80°C for about 1 to about 48 hours. A catalyst, as *N,N*-dimethylformamide, may also be used in this reaction.

When an acyl halide is employed in the coupling reaction, it is typically reacted with amine II in the presence of a suitable base to scavenge the acid generated during the reaction. Suitable bases include, by way of example, triethylamine, diisopropylethylamine, *N*-methylmorpholine. Alternatively, an excess of amine II may be used to scavenge the acid generated during the reaction.

Alternatively, the carboxylic acid of compound III can be employed in the coupling reaction. The carboxylic acid of III are usually commercially available reagents or can be prepared by conventional procedures.

The coupling reaction of carboxylic acid of III (i.e. the acyl chloride) is conducted upon using any conventional coupling reagent including, for example, carbodiimides such as dicyclohexylcarbodiimide, N-(3-dimethylaminopropyl)-N'-ethylcarbodiimide and other promoting agents, such as *N,N*-carbonyl-diimidazole or PyBOP. This reaction can be conducted with or without the use of well known additives such as *N*-hydroxysuccinimide, 1-hydroxybenzotriazole which are known to facilitate the coupling of carboxylic acids and amines.

The coupling reaction using either acid halide III or its carboxylic acid is preferably conducted at a temperature of from about 0°C to about 6°C for about 1 to about 24 hours. Typically, the reaction is conducted in an inert aprotic polar solvent such as N,N-dimethylformamide, dichloromethane, chloroform, acetonitrile, tetrahydrofuran using about 1 to about 5 molar equivalents of the amine based on the carboxylic acid or its acid halide. Upon completion of the reaction, the carboxamide IV is recovered by conventional methods including precipitation, chromatography, filtration, distillation.

The sulfonyl chorides of formula V necessary for the preparation of the sulfonylpiperidines or piperazines of formula I are prepared using conventional sulfonating methods:

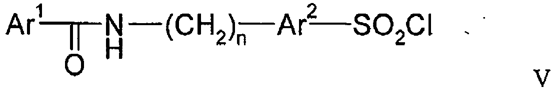

A preferred sulfonating reagent for use in this reaction is chlorosulfonic acid. Typically, the sulfonation reaction is performed by treating the carboxamide of formula (IV) with about 5 to about 10 molar equivalent of the sulfonating reagent in an inert solvent, such as dichloromethane, at a temperature ranging from about -70°C to about 50°C. Preferably, the addition of chlorosulfonic acid takes place at -70°C and leads to the formation of the intermediate sulfonic acid. Increasing the temperature to 20°C allows the formation of the sulfonyl chloride of formula V.

According to a further preferred method of preparation notably in case that the above pointed out method leading to the preliminary synthesis of sulfonyl chloride of formula V is not applicable, the sulfonyl piperidines and piperazines of this invention are prepared by the following steps:
- Protection of the amine function of compounds of formula II;
- Chlorosulfonylation of the aromatic group;
- Formation of the sulfonamide function;
- Deprotection of the protecting group;
- Acylation of the above generated free amine;

Amines of formula II are protected with a suitable protecting group of an amine moiety to provide intermediate of formula VI wherein P denotes the protecting group.

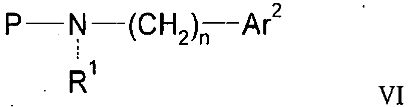

Numerous protecting groups P of the amine function as well as their introduction and removal, are well described in T.W. Greene and G.M. Wuts, Protecting groups in Organic Synthesis, Third Edition, Wiley, New York, 1998, and references cited therein. Preferred are protecting groups that are acids and bases stable and can be further removed by using metal transition complexes such as palladium complexes, for example the allylcarbamate group (Alloc) or the N,N'-bisallyl group. Another preferred protecting group is the maleimide group which is stable in a all range of experimental conditions.

The introduction of said groups can be performed by reacting the corresponding bisallylcarbonate anhydride or allylbromide or maleic anhydride in the presence of a base such as triethylamine, diisopropylethylamine, *N*-methylmorpholine in an aprotic solvent such as N,N-dimethylformamide, dichloromethane, chloroform, acetonitrile, tetrahydrofuran at a temperature ranging from about 0°C to about 80°C.

Compounds of formula VI are then sulfonated using a conventional very mild sulfonating procedure that allows the obtention of sulfonyl chloride of formula VII.

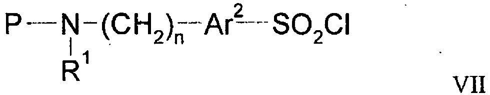

Typically, protected amine VI is treated with a base such as n-butyllithium or tert-butyllithium under an inert atmosphere, in a polar aprotic solvent such as tetrahydrofuran, ether or dioxane at a temperature ranging from -70°C to 0°C during a time ranging from 15 minutes to 4 hours. The so formed anion is then treated with SO₂Cl₂ or most preferably SO₂ by bubbling the gas into the reaction mixture at a temperature ranging from - 70°C to 20°C during a time ranging from 5 minutes to 1 hour. The sulfonate obtained is then transformed "*in situ*" to the sulfonyl chloride of formula VII by contacting with *N-*chlorosuccinimide at a temperature ranging from 0°C to 70°C.

The sulfonamide derivatives of formula I are then prepared from the corresponding above mentioned sulfonyl chloride V or VII, by reaction with a corresponding cyclic amine, e.g. a piperidine derivative of the general formula IX. 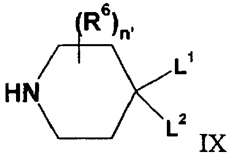 whereby L¹ and L² are as above defined.

The amines of formula IX are either commercially available compounds or compounds that can be prepared by known procedures.

The reaction is generally conducted in the presence of a base such as triethylamine, diisopropylethylamine, potassium carbonate in solvent such as N,N-dimethylformamide, dimethylsulfoxide, N-methylpyrrolidone, ethanol, acetonitrile at a temperature from about 0° to about 100°C.

The sulfonamides of formula I are readily prepared from the corresponding sulfonyl chloride V or VII, by reaction with a piperidine of general formula IX.

Piperidines of formula IX are either commercially available compounds or compounds that can be prepared by known procedures.

Typically, piperidines of type IX can be prepared using conventional methods known by one skilled in the art and described by way of examples in J. Pharm. Sci. 1972, 61, 1316; J. Heterocyclic. Chem., 1986, 23, 73; Tetrahedron Lett., 1996, 37, 1297, US 5106983, WO/9113872 and WO/9606609.

Preferred methods of obtention of piperidines of formula IX are the following :
A method in the case where n = 0 is a "Mitsunobu type" coupling between an activated aniline of type XII with mono-N-protected 4-piperidol as described in Tetrahedron Lett. 1995, 36, 6373-6374.

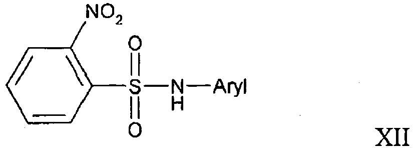

Deprotection of the sulfamino group is then carried out using thiophenol in the presence of potassium carbonate.

For L² = -NR³'C(O)R³, -NR³'C(O)NR³'R³, NR³'SO₂-R³, a preferred method of synthesis of compounds of formula IX is the reaction of commercially available N-BOC-4-aminopiperidine with respectively acyl chlorides, isocyanates and sulfonyl chloride under classical conditions very well known by one skilled in the art.

The sulfonamides of formula I are readily prepared by contacting the sulfonyl chloride V with an amine of formula IX in the presence of a suitable base to scavenge the acid generated during the reaction. Suitable bases include, by way of examples, triethylamine, diisopropylethylamine, N-methylmorpholine. The reaction is preferably conducted in solvent such as N,N-dimethyformamide, dimethylsulfoxide, N-methylpyrrolidone, ethanol, acetonitrile at a temperature from about 0° to about 100°C.

The sulfonamides of formula XIV are readily prepared by contacting the sulfonyl chloride VII with an amine of formula IX in the presence of a suitable base to scavenge the acid generated during the reaction. Suitable bases include, by way of examples, triethylamine, diisopropylethylamine, N-methylmorpholine. The reaction is preferably conducted in solvent such as N,N-dimethyformamide, dimethylsulfoxide, N-methylpyrrolidone, ethanol, acetonitrile at a temperature from about 0° to about 100°C. The use of sulfonyl chloride of type VII leads to amines that have to be deprotected using well known methods by one skilled in the art to afford amine of general formula XIV 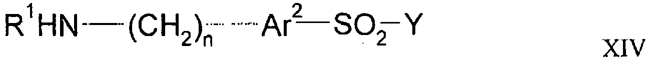 wherein R¹, Ar², Y and n are as above defined.

Derivatives of type XIV are then acylated according to described methods for the preparation of amides by condensation of amines with acid chlorides or carboxylic acids in the preferred conditions described above leading to compounds of general formula I.

If the above set out general synthetic methods are not applicable for the obtention of compounds of formula I, suitable methods of preparation known by a person skilled in the art should be used. For example, when Ar² is phenyl, one should start from commercially available 4-cyanophenyl sulfonyl chloride and applies conventional methods known by a person skilled in the art to reach sulfonamide derivatives of formula I.

A final aspect of the present invention is related to the use of the compounds according to formula I for the modulation of the JNK function, or signaling pathways, the use of said compounds for the preparation of pharmaceutical compositions for the modulation of the JNK pathway as well as the formulations containing the active compounds according to formula I. Said modulation of the JNK pathway is viewed as a suitable approach of treatment for various disorders. When employed as pharmaceuticals, the sulfonamide derivatives of the present invention are typically administered in the form of a pharmaceutical composition. Hence, pharmaceutical compositions comprising a compound of formula I and a pharmaceutically acceptable carrier, diluent or excipient therefore are also within the scope of the present invention. A person skilled in the art is aware of a whole variety of such carrier, diluent or excipient compounds suitable to formulate a pharmaceutical composition. Also, the present invention provides compounds for use as a medicament. In particular, the invention provides the compounds of formula I for use as JNK inhibitor, notably of JNK3, for the treatment of disorders of the immune as well as the neuronal system of mammals, notably of humans, either alone or in combination with other medicaments.

The compounds of the invention, together with a conventionally employed adjuvant, carrier, diluent or excipient may be placed into the form of pharmaceutical compositions and unit dosages thereof, and in such form may be employed as solids, such as tablets or filled capsules, or liquids such as solutions, suspensions, emulsions, elixirs, or capsules filled with the same, all for oral use, or in the form of sterile injectable solutions for parenteral (including subcutaneous use). Such pharmaceutical compositions and unit dosage forms thereof may comprise ingredients in conventional proportions, with or without additional active compounds or principles, and such unit dosage forms may contain any suitable effective amount of the active ingredient commensurate with the intended daily dosage range to be employed.

When employed as pharmaceuticals, the sulfonamides derivatives of this invention are typically administered in the form of a pharmaceutical composition. Such compositions can be prepared in a manner well known in the pharmaceutical art and comprise at least one active compound. Generally, the compounds of this invention are administered in a pharmaceutically effective amount. The amount of the compound actually administered will typically be determined by a physician, in the light of the relevant circumstances, including the condition to be treated, the chosen route of administration, the actual compound administered, the age, weight, and response of the individual patient, the severity of the patient's symptoms.

The pharmaceutical compositions of these inventions can be administered by a variety of routes including oral, rectal, transdermal, subcutaneous, intravenous, intramuscular, and intranasal. Depending on the intended route of delivery, the compounds are preferably formulated as either injectable or oral compositions. The compositions for oral administration can take the form of bulk liquid solutions or suspensions, or bulk powders. More commonly, however, the compositions are presented in unit dosage forms to facilitate accurate dosing. The term "unit dosage forms" refers to physically discrete units suitable as unitary dosages for human subjects and other mammals, each unit containing a predetermined quantity of active material calculated to produce the desired therapeutic effect, in association with a suitable pharmaceutical excipient. Typical unit dosage forms include prefilled, premeasured ampoules or syringes of the liquid compositions or pills, tablets, capsules in the case of solid compositions. In such compositions, the sulfonamide compound is usually a minor component (from about 0.1 to about 50% by weight or preferably from about 1 to about 40% by weight) with the remainder being various vehicles or carriers and processing aids helpful for forming the desired dosing form.

Liquid forms suitable for oral administration may include a suitable aqueous or nonaqueous vehicle with buffers, suspending and dispensing agents, colorants, flavors. Solid forms may include, for example, any of the following ingredients, or compounds of a similar nature: a binder such as microcrystalline cellulose, gum tragacanth or gelatine; an excipient such as starch or lactose, a disintegrating agent such as alginic acid, Primogel, or corn starch; a lubricant such as magnesium stearate; a glidant such as colloidal silicon dioxide; a sweetening agent such as sucrose or saccharin; or a flavoring agent such as peppermint, methyl salicylate, or orange flavoring.

Injectable compositions are typically based upon injectable sterile saline or phosphate-buffered saline or other injectable carriers known in the art. As above mentioned, the sulfonamide compound of formula I in such compositions is typically a minor component, frequently ranging between 0.05 to 10% by weight with the remainder being the injectable carrier.

The above described components for orally administered or injectable compositions are merely representative. Further materials as well as processing techniques are set out in Part 8 of Remington's Pharmaceutical Sciences, 17th Edition, 1985, Marck Publishing Company, Easton, Pennsylvania, which is incorporated herein be reference.

The compounds of this invention can also be administered in sustained release forms or from sustained release drug delivery systems. A description of representative sustained release materials can also be found in the incorporated materials in *Remington's Pharmaceutical Sciences.*

In the following the present invention shall be illustrated by means of some examples which are not construed to be viewed as limiting the scope of the invention. The HPLC, NMR and MS data provided in the examples described below were obtained as followed. The following abbreviations are hereinafter used in the accompanying examples:
min (min-ute), hr (hour), g (gram), mmol (millimole), m.p. (melting point), eq (equivalents), mL (milliliter), µL (microliters), mL (milliliters), ACN (Acetonitrile), Boc (butoxycarbonyl), CDCl₃ (deuterated chloroform), cHex (Cyclohexanes), DCM (Dichloromethane), DECP (Diethylcyanophos-phonate), DIC (Diisopropyl carbodiimide),
DMAP (4- Dimethylaminopyridine) DMF (Dimethylformamide), DMSO (Dimethylsulfoxide), DMSO-*d₆* (deuterated dimethylsul-foxide), EDC (1-(3-Dimethyl-aminopropyl)-3-ethylcarbodiimide), EtOAc (Ethyl acetate), Et₂O (Diethyl ether), Fmoc (9-fluorenylmethoxycarbonyl), HOBt (1-Hydroxybenzotriazole), K₂CO₃ (potassium carbonate), NaH (Sodium hydride), NaHCO₃ (Sodium bicarbonate), nBuLi (n-Butyllithium), TBTU (O-Benzotriazolyl-*N,N,N*'*,N*'-tetramethyluronium-tetrafluoroborate), TEA (Triethyl amine), TFA (Trifluoro-acetic acid), THF (Tetrahydrofuran), TMOF (trimethylorthoformate), MgSO₄ (Magnesium sulfate), PetEther (Petroleum ether), rt (room temperature).

### Examples

### Example 1: 5-{[(3-methoxybenzoyl)amino)methyl}-2-[(4-{3-[(trifluoromethyl)-sulfonyl]anilino}piperidin-1-yl)sulfonyl]thiophene-3-carboxylic acid (1)

### Diallyl-thiophen-2-ylmethylamine (1a)

A solution of 2-aminomethylthiophene (51.4 g, 956 mmol) and *i*-Pr₂NEt (140 g, 1081 mmol) in CH₂Cl₂ (11) was placed in a 3-1 flask equipped with a condenser and an efficient magnetic agitation. Allyl bromide (115.7 g, 454 mmol) was added, whereupon the moderately exothermic reaction spontaneously reached the reflux temperature after 2 h. The mixture was stirred overnight (16 h), washed (NaHCO₃ sat.; brine), dried (MgSO₄), and concentrated. The resulting oil was filtered over silica gel (EtOAc:hexane 1:4). The filtrate was concentrated and the filtration was repeated to afford 70.3 g (80%) of the title diallylamine as a brown-yellow oil, clean by NMR: ¹H NMR (CDCl₃) δ 7.25 (br. d, *J* = 5.9 Hz, 1H), 6.98 (br. dd, *J* = 5.1, 2.8 Hz, 1H), 6.94-6.92 (m, 1H), 5.99-5.86 (m, 2H), 5.29-5.18 (m, 4H), 3.85 (s, 2H), 3.16 (dd, *J* = 6.3, 0.9 Hz, 4H).

### 5-Diallylaminomethyl-thiophene-2-sulfonyl chloride (1b)

A solution of the allyl-protected thiophene **(1a)** (6.2 g, 32.1 mmol) in Et₂O was cooled to - 70°C by means of an acetone/dry ice bath. A solution of *t*-BuLi in pentane (21.38 ml, 1.5M, 32.1 mmol) was added over 2 min whereupon the internal temperature momentarily rose to -50°C and the mixture turned orange. After 10 min., SO₂ was bubbled for 2 min, which led to the immediate formation of a thick precipitate. The reaction was allowed to reach 0°C, and a suspension of NCS (4.63 g, 32.1 mmol) in THF (20 ml) was added, whereupon the slurry turned purple. After 45 min at r.t., the mixture was filtered over SiO₂, eluting with EtOAc. Evaporation, dilution with EtOAc:hexane 1:5 and filtration over SiO₂ gave 5.0 g (53%) of the title sulfonyl chloride **(1b)** as a pale brown oil which was used without further purification.

### N,N-Diallyl-N-{[5-(1,4-dioxa-8-azaspiro[4.5]dec-8-ylsulfonyl)thien-2-yl]methyl}amine (1c)

Procedure A (from the isolated sulfonyl chloride **(1b)**). A solution of **(1b)** (5.84 g, 20 mmol) in CHCl₃ was cooled to 0°C, and treated with 1,4-dioxa-8-azaspiro[4,5]decane (2.8 ml, 22 mmol) and Et₃N (4.2 ml, 30 mmol), and warmed to 23 °C for 10 min. Dilution with EtOAc (100 ml), standard work-up (NaHCO₃ sat.; brine; MgSO₄) and chromatography (EtOAc:cyclohexane 1:2) gave 7.57 g (95%) of the title sulfonamide as a colourless oil.

Procedure B (from **(1a)**, without isolation of the sulfonyl chloride **(1b)**). A solution of the allyl-protected thiophene **(1a)** (29.1 g, 150 mmol) in Et₂O (440 g, 617 ml) was placed in a 1-1 three-necked flask (thermometer; argon; septum or SO₂ inlet) and cooled to - 74°C by means of an acetone/dry ice bath. A solution of *t*-BuLi in pentane (100 ml, 1.5M, 150 mmol) was added over 5 min whereupon the internal temperature momentarily rose to -64°C and the mixture turned pink. After 20 min., SO₂ (20 g, 312 mmol) was bubbled over 15 min. The SO₂ consumption was best monitored by placing the SO₂ bottle on a scale during the reaction The reaction mixture, which had turned to a thick, white wax was allowed to warm to room temperature over 2h. A suspension of NCS (30 g, 226 mmol) was added, and stirring was continued overnight, whereupon the slurry turned purple. The mixture was filtered (fritted glass), and the precipitate was carefully washed with CH₂Cl₂ (2 x 300 ml). The combined organic layers were cooled to 0°C under Ar, and treated with a solution of 1,4-dioxa-8-azaspiro[4,5]decane (27.8 g, 194 mmol) and triethylamine (19.7 g, 194 mmol) in CH₂Cl₂ (200 ml). After 1 h, the mixture was washed (NaHCO₃ sat.; brine), dried (MgSO₄), and concentrated to afford 53 g (83%) of the title sulfonamide as yellow oil: ¹H NMR (CDCl₃) δ 7.36 (d, *J* = 3.8 Hz, 1H), 6.90 (br. d, *J*= 3.4 Hz, 1H), 5.92-5.79 (m, 2H), 5.33-5.16 (m, 4H), 3.93 (s, 4H), 3.78 (s, 2H), 3.21 (t, 5.7 Hz, 4H), 3.13 (d, 6.2 Hz, 4H), 1.81 (t, 5.7 Hz, 4H).

### Ethyl 5-[(diallylamino)methyl]-2-(1,4-dioxa-8-azaspiro[4.5]dec-8-ylsulfonyl)thiophene-3-carboxylate (1d)

A solution of the sulfonamide **(1c)** (3.36 g, 8.43 mmol) in THF (120 ml) was cooled to -78°C and treated with t-BuLi (7.0 ml, 1.5M in hexane, 10.5 ml). After 5 min, the mixture was canulated into a cooled (-100°C; acetone/liquid N₂) solution of ethyl chloroformate (6.45 ml, 67.5 mmol) in THF (60 ml). The reaction mixture was allowed to warm to -30°C over 2h, and then to 23°C overnight. The mixture was concentrated on a rotary evaporator and diluted with EtOAc (250 ml). Standard work-up (H₂O; brine; MgSO₄) and two chromatographies (EtOAc:cyclohexane 1:4) afforded 1.48 (37%) of the title ethyl ester: ¹H NMR (*DMSO*-*d6*) δ 7.36 (d, 1H), 5.98-5.82 (m, 2H), 5.32-5.17 (m, 4H), 4.33 (q, *J* = 7.1 Hz, 2H), 3.92 (s, 4H), 3.85 (s, 2H), 3.32 (dd, *J* ≈ 6.0, 5.0 Hz, 4H), 3.17 (d, *J* = 6.0 Hz, 4H), 1.74 dd, *J* ≈ 6.0, 5.0 Hz, 4H), 1.33 (t, *J* = 7.2 Hz, 3H).

### Ethyl 2-(1,4-dioxa-8-azaspiro[4.5]dec-8-ylsulfonyl)-5-{[(3-methoxybenzoyl)amino]-methyl}thiophene-3-carboxylate (1e)

A solution of the ethyl ester **(1d)** (1.47 g, 3.12 mmol) and NDMBA (1.07 g, 6.87 mmol) in CH₂Cl₂ (30 ml) was degassed by bubbling argon and sonicating. Then, Pd(PPh₃)₄ (216 mg, 0.187 mmol) was added and the mixture was stirred at 23°C. After 2h, the mixture was cooled to -50°C, treated with Et₃N (525 ul, 3.76 mmol) and 3-(methoxy)-benzoyl chloride (300ul, 2.13 mmol), and warmed to r.t. over 30 min. Dilution with EtOAc, standard work-up (H₂O; NaHCO₃ sat.; brine; MgSO₄) and chromatography (EtOAc:cyclohexane 1:1) afforded 1.0 g (61%) of the title 3-methoxybenzamide: ¹H NMR (DMSO-*d6*) 9.29 (t, *J* = 5.8 Hz, 1H), 7.49-7.34 (m, 4H), 7.12 (ddd, *J* = 7.9, 2.6, 1.0 Hz, 1H), 4.66 (d, *J* = 5.7 Hz, 2H), 4.27 (q, *J* = 7.2 Hz, 2H), 3.84 (s, 4H), 3.80 (s, 3H), 3.24 (dd, *J ≈* 6.0, 5.0 Hz, 4H), 1.67 (dd, *J ≈* 6.0, 5.0 Hz, 4H), 1.26 (t, *J* = 7.0 Hz, 3H). M/Z APCI: 525 (M + 1), 523 (M-1).

### Ethyl 5-{[(3-methoxybenzoyl)amino]methyl}-2-[(4-oxopiperidin-1-yl)sulfonyl]-thiophene-3-carboxylate (1f)

A solution of the spiroketal **(1e)** (500 mg, 0.953 mmol) in acetone (5 ml) was treated with HCl 1N (2.5 ml) for 18 h at 48°C. Dilution with EtOAc and standard work-up (H₂O; NaHCO₃ sat.; brine; MgSO₄) gave 425 mg of a 9:1 mixture of the desired title ketone (83%) and of unreacted starting material (9%) (single spot by TLC). ¹H NMR (CDCl₃) 7.37-7.35 (m, 1H), 7.33-7.29 (m, 3H), 7.05 (ddd, *J* = 7.7, 2.6, 1.7 Hz, 1H), 6.81 (t, *J* = 5.8 Hz, 1H), 4.74 (d, *J* = 6.1 Hz, 2H), 4.31 (q, *J* = 7.1 Hz, 2H), 3.83 (s, 3H), 3.70 (t, *J* = 6.1 Hz, 4H), 2.52 (t, *J* = 6.2 Hz, 4H), 1.34 (t, *J* = 7.1 Hz, 3H). M/Z APCI: 481 (M + 1), 479 (M-1).

### Ethyl 5-{[(3-methoxybenzoyl)amino]methyl}-2-[(4-{3-[(trifluoromethyl)sulfonyl]-anilino}piperidin-1-yl)sulfonyl]thiohene-3-carboxylate (1g)

A suspension of the crude ketone **(1f)** (425 mg, 0.803 mmol), 3-(trifluoromethylsulfonyl)-aniline (287 mg, 1.27 mmol), and 3Ǻ powdered MS (3 g) in dry tetrachloroethylene (15 ml) was heated to reflux for 17h under strictly anhydrous conditions. The mixture was cooled to 23°C, and finely powdered NaBH(OAc)₃ (1.2 g) was added. The stirring was continued for 2.5 d.. Dilution with EtOAc, standard work-up (NaHCO₃ sat.; brine; MSO₄) and chromatography (EtOAc:cyclohexane 1:1.5 → 2:1) afforded 167 mg (35%) of a mixture of the starting ketone **(1f)** and spiroketal **(1e),** and 216 mg (39%) of the title anilinopiperidine. ¹H NMR (DMSO-d6) 9.16 (t, *J* = 5.8 Hz, 1H), 7.38-7.23 (m, 5H), 6.97-7.08 (m, 4H), 6.42 (d, *J* = 7.9 Hz, 1H), 4.53 (d, *J* = 5.7 Hz, 2H), 4.15 (q, *J* = 7.0 Hz, 2H), 3.68 (s, 3H), 3.53 (dm, *J* = 10.4 Hz, 2H), 3.60-3.43 (m, 1H), 2.81 (br. t, *J* = 10.6 Hz, 2H), 1.84 (dm, *J* ≈ 11.3 Hz, 2H), 1.35-1.20 (m, 2H), 1.15 (t, *J* = 7.0 Hz, 3H). M/Z APCI: 690 (M + 1), 688 (M-1).

### 5- {[(3-Methoxybenzoyl)amino]methyl}-2-[(4-{3-[(trifluoromethyl)sulfonyl]anilino}-piperidin-1-yl)sulfonyl]thiophene-3-carboxylic acid (1)

A solution of the ethyl ester **(1g)** (40 mg, 0.058 mmol) in MeOH (4 ml) was treated with NaOH 2M (0.8 ml) for 2 h at 45°C. The mixture was diluted with EtOAc, washed (NH₄Cl aq.; H₂O; brine), dried (MgSO₄), concentrated to 2 ml, and filtered over celite, eluting with EtOAc. Evaporation gave 40 mg (96%) of the title acid. M/Z APCI : 662 (M+1), 660 (M-1), 616 (M-CO₂-1). Anal. HPLC: R.t = 6.55 min (method a).

The following compounds (designated as Example No.) was prepared according to the above described procedure by replacing 3-(trifluoromethylsulfonyl)-aniline with the appropriate amine in the reductive amination step.

The following table provides HPLC data and mass spectroscopy data of the mentioned examples (HPLC conditions: C8 Symmetry a- MeCN, 0.09%TFA, 0 to 100% (10min); Mass spectrum APCI).

| Example | Name | Rt HPLC | Purity (%) | Gradient HPLC | Mass M+1 | Mass M-1 |
|---|---|---|---|---|---|---|
| **2** | 5-{[(3-methoxybenzoyl)amino]methyl}-2-{[4-(octylamino)piperidin-1-yl]sulfonyl}thiophene-3-carboxylic acid | 4.58 | 90.1 | a | 567 | 565 |
| **3** | 2-{[4-(hexylamino)-1-piperidinyl]sulfonyl}-5-{[(3-methoxybenzoyl)amino]methyl}-3-thiophenecarboxylic acid | 4.04 | 98 | a | 538 | 536 |
| **4** | 5-{[(3-methoxybenzoyl)amino]methyl}-2-[(4-{[4-(trifluoromethyl)benzyl]amino}-1-piperidinyl)sulfonyl]-3-thiophenecarboxylic acid | 4.20 | 95 | a | - | 610 |

### Example 5: N-(2-hydroxyethyl)-5-{[(3-methoxybenzoyl)amino]methyl}-2-[(4-{3-[(trifluoromethyl)sulfonyl]anilino}piperidin-1-yl)sulfonyl]thiophene-3-carboxamide

A solution of the ethyl ester **(1g)** (10 mg, 0.015 mmol) and ethanolamine (0.1 ml) in MeOH (1 ml) was heated to reflux for 8 h and concentrated to dryness to afford the title amide 5 in nearly quantitative yield. M/Z APCI : 705 (M+1), 703 (M-1). Anal. HPLC: R.t = 6.14 min (method a).

The following compounds (designated as Example No.) were prepared according to the above described procedure (example 2) by replacing ethanolamine with hydrazine, aqueous ammonia or N,N'-dimethylaminoethylene diamine.

The following table provides HPLC data and mass spectroscopy data of the mentioned examples.

| **Example** | **Name** | **Rt HPLC** | **Purity** | **Gradient HPLC** | **Mass M+1** | **Mass M-1** |
|---|---|---|---|---|---|---|
| **6** | N-({4-(hydrazinocarbonyl)-5-[(4-{3-[(trifluoro-methyl)sulfonyl]anilino}piperidin-1-yl)sulfonyl]-thien-2-yl}methyl)-3-methoxybenzamide | 5.67 | 79.0 | a | 520 | 518 |
| **7** | 5-{[(3-methoxybenzoyl)amino]methyl}-2-[(4-{3-[(trifluoromethyl)sulfonyl]anilino}piperidin-1-yl)sulfonyl]thiophene-3-carboxamide | 5.63 | 84.4 | a | 661 | 659 |
| **8** | N-[2-(dimethylamino)ethyl]-5-{[(3-methoxy-benzoyl)amino]methyl}-2-[(4-{3-[(trifluoro-methyl)sulfonyl]anilino}piperidin-1-yl)sulfonyl]-thiophene-3-carboxamide | 4.85 | 98.0 | a | 732 | 730 |

### Example 9: N-({4-(hydroxymethyl)-5-[(4-{3-[(trifluoromethyl)sulfonyl]anilino}-piperidin-1-yl)sulfonyl]thien-2-yl}methyl)-3-methoxybenzamide (9)

A solution of the carboxylic acid (19 mg, 0.029 mmol) was dissolved in borane-THF complex (1M in THF, 1ml, 1 mmol), and the solution was stirred for 30 min at 23°C. The reaction was quenched with water (1 ml, fizz!), diluted with EtOAc (10 ml), dried (MgSO₄), concentrated, and chromatographed (EtOAc:cyclohexane 1:2 → 2:1) to give 11.1 mg (60%) of the title alcohol. M/Z APCI : 648 (M+1), 646 (M-1). Anal. HPLC: R.t = 6.43 min (method a).

### Example 10 : Preparation of a pharmaceutical formulation

The following formulation examples illustrate representative pharmaceutical compositions according to the present invention being not restricted thereto.

### Formulation 1 - Tablets

A sulfonamide compound of formula I is admixed as a dry powder with a dry gelatin binder in an approximate 1:2 weight ration. A minor amount of magnesium stearate is added as a lubricant. The mixture is formed into 240-270 mg tablets (80-90 mg of active sulfonamide compound per tablet) in a tablet press.

### Formulation 2 - Capsules

A sulfonamide compound of formula I is admixed as a dry powder with a starch diluent in an approximate 1:1 weight ratio. The mixture is filled into 250 mg capsules (125 mg of active sulfonamide compound per capsule).

### Formulation 3 - Liquid

A sulfonamide compound of formula I (1250 mg), sucrose (1.75 g) and xanthan gum (4 mg) are blended, passed through a No. 10 mesh U.S. sieve, and then mixed with a previously prepared solution of microcrystalline cellulose and sodium carboxymethyl cellulose (11:89, 50 mg) in water. Sodium benzoate (10 mg), flavor, and color are diluted with water and added with stirring. Sufficient water is then added to produce a total volume of 5 mL.

### Formulation 4 - Tablets

A sulfonamide compound of formula I is admixed as a dry powder with a dry gelatin binder in an approximate 1:2 weight ratio. A minor amount of magnesium stearate is added as a lubricant. The mixture is formed into 450-900 mg tablets (150-300 mg of active sulfonamide compound) in a tablet press.

### Formulation 5 - Injection

A sulfonamide compound of formula I is dissolved in a buffered sterile saline injectable aqueous medium to a concentration of approximately 5 mg/ml.

### Example 11 : Biological assays

### Biological Results

The activities of the sulfonamide derivatives claimed in the formula I were assessed using the above described *in vitro* and *in vivo* biological assays.

### JNK2 and -3 in vitro assays:

The phosphorylation of c-jun by JNK2 or JNK3 may be followed by monitoring the incorporation of ³³P into c-jun following the protocol below. The inhibitory activity of the compounds according to formula I, in respect of c-jun phosphorylation through JNK, is determined by calculating the phosphorylation activity of a JNK in the presence or absence of the test compounds according to formula I.

JNK3 and/or -2 assays are performed in 96 well MTT plates: incubation of 0.5 µg of recombinant, pre-activated GST-JNK3 or GST-JNK2 with 1 µg of recombinant, biotinylated GST-c-Jun and 2 µM ³³γ-ATP (2 nCi/µl), in the presence or absence of compounds according to formula I and in a reaction volume of 50 µl containing 50 mM Tris-HCl, pH 8.0; 10 mM MgCl₂; 1 mM Dithiothreitol, and 100 µM NaVO₄. The incubation is performed for 120 min. at R.T and stopped upon addition of 200 µl of a solution containing 250 µg of Streptavidine-coated SPA beads (Amersham, Inc.)*, 5 mM EDTA, 0.1% Triton X-100 and 50 µM ATP, in phosphate saline buffer.

After incubation for 60 minutes at RT, beads are sedimented by centrifugation at 1500 x g for 5 minutes, resuspended in 200 µl of PBS containing 5 mM EDTA, 0.1% Triton X-100 and 50 µM ATP and the radioactivity measured in a scintillation β counter, following sedimentation of the beads as described above. By replacing biotinylated GST-c Jun with biotinylated GST-₁ATF₂ or biotinylated myelin basic protein, this assay may also be used to measure inhibition of pre-activated p38 and ERK MAP Kinases, respectively.

| *Example No* | *JNK3 IC₅₀ (µ M)* |
|---|---|
| **1** | < 0.1 |
| **4** | < 0.1 |
| **6** | < 0.1 |

The values indicated in respect of refer to the IC₅₀ (µM), i.e. the amount necessary to achieve 50% inhibition of said target. Said values show a considerable potency of the sulfonamide compounds with regard to JNK3.

The tested compounds according to formula I display an inhibition (IC₅₀) with regard to JNK3 of less than 0.1 µM, more preferred equal or less than 0.02 µM.

### Sympathetic Neuron Culture and Survival Assay

### The ability of the compounds according to formula I to increase the survival rate of neuronal cells having been induced to cell death was assessed using the following protocol

Sympathetic neurons from superior cervical ganglia (SCG) of new-born rats (p4) are dissociated in dispase, plated at a density of 10⁴ cells/cm² in 48 well MTT plates coated with rat tail collagen, and cultured in Leibowitz medium containing 5% rat serum, 0.75 µg/mL NGF 7S (Boehringer Mannheim Corp., Indianapolis, IN.) and arabinosine 10⁵M. Cell death is induced at day 4 after plating by exposing the culture to medium containing 10 µg/mL of anti NGF anti-body (Boehringer Mannheim Corp., Indianapolis, IN.) and no NGF or arabinosine, in the presence or absence of sulfonamide inhibitors. 24 hours after cell death induction, determination of cell viability is performed by incubation of the culture for 1 hour, at 37°C in 0.5 mg/mL of 3-(4,5-dimethylthiazol-2-yl)2,5 diphenyl tetrazolium bromide (MTT). After incubation in MTT cells are resuspended in DMSO, transferred to a 96 MTT plate and cell viability is evaluated by measuring optical density at 590 nm.

### Il-2 Release Assay:

### The ability of the compounds according to formula I to modulate the inflammatory response by inhibiting the release of IL-2 was assessed using the following protocol

JNK pathway activation triggers the production of inflammatory cytokines such as IL-2. JNK can be activated by external stimuli such as PMA and Ionomycine and IL-2 production can be measured via an IL-2 ELISA test. Comparative measurements with and without the compounds of the invention according to the following protocol measure the ability of the compounds to prevent to stress-mediated IL-2 release.

Jurkat cells, a human T cell leukemia cell line (American Type Culture Collection # TIB 152) were cultured in RPMI 1640 medium (Gibco, BRL) supplemented with 10% of heat-activated fetal calf serum (FCS), Glutamine and Penstrep. The cell suspension in the medium is diluted to give 2.10⁶ cells/mL. The cells were plated (2.10⁵ cells/well) on a 96-well plate containing different concentrations of a compound according to formula I (final concentration of compounds, 10, 3, 1, 0.3, 0.1 µM). This mixture is incubated 30 minutes at 37°C in a humidified CO₂ atmosphere. Cells were then treated with 10 µl PMA (Phorbolmyristate-13 Acetate-12) + Ionomycine (0.1 µM and 1 µM final concentration) in all wells except negative control. In wells without compounds, 10 µl of RPMI 2% DMSO (=0.1% final) is added. Cells are incubated 24 hours at 37°C and then the supernatant harvested (freeze at -20°C if not used the same day) prior to performing IL-2 ELISA test on the supernatant.

### IL-2 ELISA Assay:

IL-2 release into the medium by (PMA + Iononomycin)-stimulated Jurkat cells, in presence or absence of test compounds may be assayed by ELISA. Following the procedure described below.

Monoclonal anti-human IL-2 antibody (MAB602) (capture), biotinylated anti-human IL-2 antibody (BAF202) (detection) and recombinant human IL-2 (202-IL-010) (standard) from From R&D Systems are used.

### Plate preparation

100 µl capture antibody diluted in PBS at 5 µg/mL (PBS- Tween 0.05%) are transferred into a 96 well ELISA plate and incubated overnight at room temperature.

Each well is aspirated and washed 3 times with wash buffer (PBS- Tween 0.05%). After the last wash, the plate is damped.

### Assay procedure

1. 100 µl of sample or standard are added (2000, 1000, 500, 250, 125, 62.5, 31.25pg/mL) and incubated 2 hours at room temperature.
2. 3-time-wash
3. 100 µl of biotinylated anti-human IL-2 at 12.5 ng/mL are added and incubated 2 hours at room temperature.
4. 3-time-wash
5. 100 µl streptavidin-HRP (Zymed #43-4323) at 1:10'000 are added and incubate 30 minutes at room temperature.
6. 3-time-wash
7. 100 µl substrate solution (citric acid/ Na₂HPO₄ (1:1) + H₂O₂ 1:2000 + OPD) are added and incubated 20-30 minutes at room temperature.
8. 50 µl of stop solution (H₂SO₄ 20%) are added to each well.
9. Optical density is measured using a microtiter plate reader set to 450 nm with correction at 570 nm.

### C-Jun Reporter Assay

The phosphorylation of the transcriptional factor, c-jun, by JNK in the MAP kinase signal transduction pathway can be followed via a trans-reporting system such as the commercially available PathDetect ® **(32).**

Inhibition of phosphorylation by compounds according to formula I can then be assessed.

A trans-reporting system allows one to follow, via Luciferase activity, the activation status of a fusion trans-activator protein. The trans-activator protein consists of the activation domain of the transcriptional factor of interest (c-jun) fused with a yeast transcriptional activator, GAL4 DNA binding domain (dbd). The GAL4 dbd has the advantage that no known mammalian transcriptional factors can bind to it and therefore the background noise of the assay is very low.

In the present case, Hela luciferase reporter-c-Jun (HLR-c-Jun) cell lines which constitutively express GAL4-cJun were used.

The MEEK-1 gene was inserted. MEKK-1 is a MAPKKK which triggers the activation of JNK. Expression of wild type MEKK-1 is sufficient for JNK activation **(33).**

Once, JNK is activated it can induce the phosphorylation of the c-jun domain of the fusion trans-activator protein (GAL4dbd -cJun) which forms a dimer. The dimer is then is able to bind to a GAL4 upstream activating sequence (GAL4 UAS) of the reporter which activates Luciferase expression.

Luciferase expression is detected by luminescence using a simple assay such as Dual-Luciferase ® Reporter Assay System **(34)** in which Renilla is used as a "control reporter".

Inhibition of JNK is observed as a decrease in Luciferase expression and detected by a decrease in luminescence.

### Cell culture

HLR-c-Jun cells are cultured in DMEM High Glc supplemented with 10% FCS (Sigma), 2mM Glutamine (Gibco), P/S, Hygromycin b 100µg/mL and G418 250µg/mL.

### Cell culture preparation

### Cell Banks

The cells are stored frozen in cryotubes under liquid nitrogen, as 1.8 mL volumes of cell suspension in culture medium containing 10% dimethyl sulfoxide.

### Cell culture thawing

When necessary, frozen vials of cells are thawed rapidly at 37°C in a water bath by gently swirling up to semi-complete thawing. Then the cell suspension is added to 10 mL of culture medium and then centrifuged for 5 minutes at 1200 rpm. The supernatant is removed and the cell pellet reconstituted in the medium. The flasks are incubated at 37° C in an atmosphere of % CO₂.

### Cell passage

The cells are serially sub-cultured (passaged) when 80% confluent monolayers have been obtained.

The medium of each flask is removed and the monolayer is washed with 10-15 mL of phosphate buffer solution (PBS).

Trypsin-EDTA solution is added to the cell monolayer, incubated at 37° C and tapped gently at intervals to dislodge the cells. Complete detachment and disaggregation of the cell monolayer is confirmed by microscopy examination. The cells are then re-suspended in 10 mL of complete medium and centrifuged for 5 minutes at 1200 rpm. The supernatants are discarded, the cells are re-suspended in culture medium and diluted 1/5 in 175 cm² flasks.

### Day 0 morning

### Prepare cells for transfections

The cells of near-confluent cultures are detached and disaggregated by treatment with trypsin as described above.

The cells are re-suspended in culture medium and counted.

The cell suspensions are diluted with medium to give about 3.5×10⁶ cells/mL and 1mL µl of cell suspension are put onto 2 10cm culture dishes containing 9 mL of culture medium.

The plates are incubated at 37° C in a humidified atmosphere of 5 % CO₂ in air.

### Day 0 evening

### Transfections

| | |
|---|---|
| Control | :0.2µg pTK Renilla, 5.8µg pBluescript KS, 500µl OPTIMEM (GIBCO), 18µl Fugene 6. |
| Induced | :0.1 µg pMEKK1, 0.2µg pTK Renilla, 5.7µg pBluescript KS, 500µl OPTIMEM (GIBCO), 18µl Fugene 6 30' RT. |

The transfection mixture is added to the plated cells. The plates are incubated over night at 37° C in a humidified atmosphere of 5 % CO₂ in air.

### Day 1

A 96 wells plate (100 µl of culture medium per well) is prepared.

Negative control (vehicle): 2µl of DMSO is added to the 100µl (in triplicate). 2 µl of compound according to formula I stock dilutions (3, 1 and 0.1mM in 100% DMSO) are added to the 100µl (in triplicate).

The transfected cells are trypsinised and re-suspended in 12 mL of culture medium. 100µl of the dilution are added to each of the 96 wells plate.

The plate is incubated over night at 37° C in a humidified atmosphere of 5 % CO₂ in air.

### Day 2

Test procedure: Dual-Luciferase ® Reporter Assay System **(34).**

The medium is removed from the plate and the cells are washed two times with 100µl PBS. Lysis reagent is applied (Passive Lysis Buffer, PLB). Into each culture well 5µl of 1X PLB are dispensed. The culture plates are placed on a rocking platform or orbital shaker with gentle rocking/shaking to ensure complete coverage of the cell monolayer with 1Y PLB. The culture plates are rocked at room temperature for 15 minutes. 20 µl of the lysate are transferred into a white opaque 96 well plate. The luminometer reading is recorded.

- 50 µl of Luciferase Assay Reagent II are injected and readings are recorded at 5 and 10 minutes.

50µl of Stop & Glo ® Reagent are injected and readings are recorded at 5 and 10 minutes.

The relative luminescence is then measured: RLU Luciferase/RLU Renilla.

### LPS induced endotoxin shock in mice

### The ability of the JNK inhibitors described in formula I to significantly reduce the level of inflammatory cytokines induced by LPS challenge was assessed using the following protocol:

Endotoxins are the lipopolysaccharides (LPS) constituents of the outer membrane of Gram negative bacteria. Response to LPS has been shown to involve the activation of different cell populations and to lead to the expression of various inflammatory cytokines that include tumor necrosis factor-alpha (TNFα) and interferon gamma (IFN-γ).

As LPS is known to stimulate the activation of various MAP kinase pathways, including JNK (35), the ability of JNK inhibitors can be tested after the JNK signaling pathway has been switched on by a LPS challenge.

The activity as JNK inhibitors of compounds of formula may be assessed after a LPS challenge using the following protocol:
LPS (S. abortus-Galanos Lab.-) is injected (200 µg/kg, i.v.) to Male C57BL/6 mice to induce endotoxin shock. Compounds according to formula I (0.1, 1, 10 mg/kg) or NaCl (200uM) are injected intravenously (10 mL/kg) 15 min before the LPS challenge. Heparinized blood was obtained from the orbital sinus at different time points after the LPS challenge, and the blood was centrifuged at 9'000 rpm for 10 min at 4° C to collect supernatant. Measurement of cytokines production such as TNFα and IFNγ by mouse is performed with an ELISA kit such as Duoset ® DY410 for TNFα and DY 485 for IFN γ. Other ELISA assays such as described in **(36)** can be used.

### Global Ischemia in Gerbils

### The ability of the JNK inhibitors described in formula I to protect cell death during a stroke event was assessed using the following protocol:

The gerbil bilateral carotid occlusion is a well-described animal model of acute ischemic stroke and involves relatively easy surgical techniques.

The neuronal degeneration in the hippocampus develops over several days and is often referred as "delayed neuronal death". In addition, the neurodegeneration observed histologically is obvious and easily quantified **(37).** Furthermore, the histopathology seen in the gerbil is similar to that observed in the hippocampal CA1 region of the human brain following a cardiac arrest. Behavior observations, such as memory tests, could even be performed in the case of gerbils. This kind of tests for appreciation of the degree of recovery is not easily manageable in other models such as in rat whose learning abilities are much poorer **(38).**

The neuroprotective effect according to formula I to protect may be assessed using the gerbil global ischemia model and such a protocol:

### -1- METHOD

* Surgery
   - Anesthesia with isoflurane (0.5-4%).
   - The common carotid arteries (left and right) are freed from tissue.
   - Occlusion of the arteries using Bulldog microclamps during 5 min.
   - Removal of clamps (reperfusion)
   - Stabulation of the animals under heating lamp until awake.
   - Stabulation of the animals in the animalry in individual cages.
* Sacrifice of the animals
   - 7 days after ischemia (Decapitation or overdose of pentobarbital).
   - Sampling of the brain.
* Histological parameters
   - Freezing of the brain in isopentane (-20°C)
   - Slicing of the hippocampus using a cryo-microtome (20 µm).
   - Staining with cresyl violet method
   - Evaluation of the lesions (in CA1/CA2 subfields of the hippocampus) by a modified Gerhard & Boast score **(39).**

### -2- TREATMENT

- Administration of the compound according to formula I or the vehicle: 15 min, 24 hours and 48 hours after reperfusion (5-10 min after the recovery of the anesthesia).
- Standard protocol
   50 animals : 5 groups of 8 (group A : control, groups B-D : test article at 3 doses and group
   E : reference compound (Orotic acid 3x300 mg/kg, *ip*).

### Solubility of compounds of formula (I)

The compounds have been assessed in respect of their solubility in water, at a pH of 7.4 at room temeprature. In general the solubility of compounds of formula (I) is in a range of at least 50 µg/mL solvent, more preferably of at least 100 µg/mL solvent. Compound 1 displays a solubility at r.t. at pH 7.4 of 0.18mg/ml.

### References:

**1.** Davis, Roger J., Signal Transduction by the JNK Group of MAP Kinases. Cell, 2000, 103: 239-252.
**2.** Chen, Yi-Rong and Tan, Tse-Hua. The c-Jun N-terminal kinase pathway and apoptotic signaling. International Journal of Oncology, 2000, 16: 651-662
**3.** Ip, YT. and Davis RJ, Signal transduction by the c-Jun N-terminal kinase (JNK) from c-Jun N-terminal kinase (JNK) from inflammation to development Curr Opin Cell Biol 1998,10:205-219.
**4.** Leppä, S. and Bohmann D., Diverse functions of JNK signalling and c-Jun in stress response and apoptosis, Oncogene 1999, 18(45):6158-6162.
**5.** Minden, A. and Karin M.. Regulation and function if the JNK subgroup of MAP kinases. Biochim Biophys Acta 1997,1333:F85-F104*.*
**6.** Whitmarsh, A.J., and Davis. R.J. Transcription factor AP-1: regulation by mitogen activated protein kinases signal transduction pathways. J. Mol, Med. 1996, 77, 2360-2371.
**7.** Gupta, S. et al., Selective interaction of JNK protein kinase isoforms with transcription factors. The EMBO Journal, 1996, 158(11): 2760-2770.
**8.** Derek D. et al., Absence of excitotoxicity- induced apoptosis in the hippocampus of mice lacking the Junk3 gene. Nature 1997, 389:865-876.
**9.** Martin, Loel H. et al., Developmental expression in the mouse nervous system of the p493F12 SAP kinase. Molecular Brain Research, 1996, 35: 47-57.
**10.** Kumagae, Y. et al., Human c-Jun N-terminal kinase expression and activation in the nervous system, Molecular Brain Research 1999, 67: 10-17
**11.** Dumitru, Calin D. et al.. TNF-alpha induction by LPS is regulated posttranscriptionally via a Tp12/ERK-dependent pathway. Cell 2000, 103: 1071-1083.
**12.** Han, Z. et al., C-Jun N-terminal kinases is required for metalloproteinase expression and joint destruction in inflammatory arthritis. The Journal of Clinical Investigation 2001, 108 (1):73-81.
**13.** Nishina, H., et al.. Impaired CD28-mediated interleukin 2 production and proliferation in stress kinase SAPK/ERK1 kinase (SEK1)/mitogen-activated protein kinase kinase 4 (MKK4)-deficient T lymphocytes. Journal of Experimental Medicine 1997, 186(6): 941-953.
**14.** Kempiak, Stephan J. et al.. The Jun Kinase Cascade is responsible for activating the CD28 Response element of the IL-2 Promoter: proof of cross-talk with the IKB Kinase Cascade, The Journal of Immunology, 1999, 162: 3176-3187.
**15.** De la Monte, S. M. et al., Oxygen free radical injury is sufficient to cause some Alzheimer-type molecular abnormalities in human CNS neuronal cells. J. Alzheimer's Dis. 2000, 2(3-4): 261-281.
**16.** Zhu,X, Activation and redistribution of c-Jun N-terminal kinase/stress activated protein kinase in degenerating neurons in Alzheimer's disease. Journal of Neuro-chemistry 2001, 76: 435-441
**17.** Force, T. et al., Stress-Activated Protein Kinases in cardiovascular Disease. Circulation Research. 1996, 78:947-953.
**18.** Kim, S. et al., Angiotensin blockade inhibits activation of mitogen-activated Protein Kinases in Rat balloon-injured artery. Circulation 1998, 97:1731-1737.
**19.** Xu, Q. et al., Acute Hypertension Activates Mitogen-activated Protein Kinases in Arterial Wall. The Journal of Clinical Investigation 1996, 97 (2):508-514.
**20.** Bogoyevitch, M.A. et al., Stimulation of the stress-activated mitogen-activated protein kinase subfamilies in perfused heart. Circulation Research. 1996, 79:162-173.
**21.** Pombo, CM. et al., The stress-activated protein kinases are major c-Jun amino-terminal kinases activated by ischemia and reperfusion, J. Biol. Chem. 1994, 269 (42): 26546-26551.
**22.** Onishi, I. et al., Activation of c-Jun N-terminal kinase during ischemia and reperfusion in mouse liver, FEBS Letters 1997, 420: 201-204
**23.** Safirstein, R., Renal stress response and acute renal failure Adv. Ren. Replace Ther. 1997, 4(2 Suppl 1): 38-42.
**24.** Butterfield, L. et al., C-Jun NH2-terminal kinase regulation of the apoptotic response of small cell lung cancer cells to ultraviolet. The Journal of Biological Chemistry 1997, 272 (15) : 10110-10116.
**25.** Hu, M. et al., JNK1, JNK2 and JNK3 are p53 N-terminal serine 34 kinases, Oncogene 1997, 15: 2277-2287.
**26.** Xu, X. et al., Constitutively activated JNK is associated with HTLV-1 mediated tumorigenesis, Oncogene 1996, 13: 135-142.
**27.** Chen YR and Tan TH, The c-Jun N-terminal kinase pathway and apoptotic signaling, Int. J. Oncol. 2000, 16(4):651-62.
**28.** Harding, T.C. et al, Inhibition of JNK by overexpression of the JNK binding domain of JIP-1 prevents apoptosis in sympathetic neurons, The Journal Of Biological Chemistry 2001, 276(7):4531-4534.
**29.** Gennaro, A.R. et al., Remington's Pharmaceutical Sciences. 18th ed. Easton: The Mack Publishing Company, 1995.
**30. Green TW and Wuts PG, 1999,** 3^{rd} Edition, Wiley Ed.
**31.** Abdel-Magid AF et al., Reductive amination of aldehvdes and ketones with sodium triacetoxyborohydride. Studies on direct and indirect reductive amination procedures, Journal of organic Chemistry 1996, 61, 3849-62.
**32.** Xu, L. et al., Assess the in-vivo activation of signal transduction pathways with Pathdetect ® reporting systems, Strategies 2001, 14 (1): 17-19.
**33.** Xu, S. et al., Cloning of rat MEK kinase 1 cDNA reveals an endogenous membrane-associated 195-kDa protein with a large regulatory domain, Proc. Natl. Acad. Sci. USA 1996, 93:5291-5295.
**34. Patent Number** US 5,744,320**;** Promega Corporation; April 28, 1998
**35.** Guha, M. and Mackman, N., LPs induction of gene expression in human monocytes, Cellular Signalling 2001, 13: 85 -94.
**36.** Fomsgaard, A. et al., Quantification and biological activities of native tumour necrosis factor from LPS-stimulated human monocytes, APMIS 1990, 98(6): 529-34.
**37.** Hunter J.L. et al., Animal models of acute ischaemic stroke: can they predict clinically successful neuroprotective drugs? TIPS 1995, 16:123-128.
**38.** Block, F., Global Ischemia And Behavioural Deficits, Progress in Neurobiology 1999, 58: 279-295.
**39.** Gerhard SC and Boast CA, Behavioral Neuroscience 1988, 102: 301-303.

## Claims

1. Hydrophilic sulfonamide derivatives according to formula I 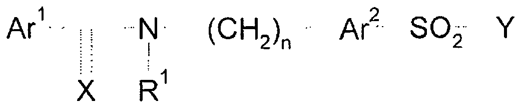 with its geometrical isomers, in an optically active form as enantiomers, diastereomers,
as well as in the form of racemates and the pharmaceutically acceptable salts thereof, wherein
Ar¹ is phenyl optionally substituted by -OR wherein R is C₁-C₆alkyl;
Ar² is a thienyl) group carrying at least one hydrophilic substituent wherein the hydrophilic substituent is -COOR³, -CONR³R^{3'}, OH, C₁-C₄alkyl substituted with OH or an amino group, a hydrazido carbonyl group, a sulfate, a sulfonate, an amine or an ammonium salt;
X is 0 or S;
R' is hydrogen or a C₁-C₆-alkyl group;
n is an integer from 1 to 3;
Y has the general formula 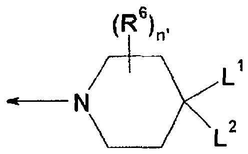 whereby, L¹ and L² are independently selected from the group consisting of H. - NR^{3'}R³, -NR³'C(O)R³, -NR^{3'}C(O)NR^{3'}R³ -(SO)R³, -(SO₂)R₃, -NSO₂R³,-SO₂NR^{3'}R³,
with R³, R^{3'}being substituents independently selected from the group consisting of H, C₁-C₆-alkyl, C₂-C₆-alkenyl, aryl being phenyl, aryl-C₁-C₆alkyl being phenyl-C₁-C₆-alkyl,
said aryl group being optionally substituted by halogen, hydroxy, nitro, sulfonyl;
R⁶ is selected from the group consisting of hydrogen, C₁-C₆-alkyl, C₁-C₆-alkoxy, OH, halogen, nitro, cyano, sulfonyl, oxo (=0), and
n' is an integer from 0 to 4.

2. A sulfonamide derivative according to claim 1, wherein Ar¹ is a phenyl group optionally substituted by -OR wherein R is C1-C6alkyl, X is 0, R1 is hydrogen, n is 1, Ar2 is a thienyl group carrying one group selected from -COOR³,-CONR³R^{3'}, OH, a C₁-C₄ alkyl substituted with an OH or amino group, a hydrazido carbonyl group.

3. A sulfonamide derivative according to claim 2, wherein Y is 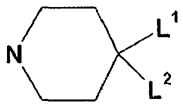 whereby L² is H, L¹ is -NHR³ with R³ being a substituent selected from the group consisting of C₁-C₆-alkyl, an aryl being phenyl, aryl-C₁-C₆-alkyl being phenyl-C₁-C₆-alkyl
said aryl group being optionally substituted by halogen, hydroxy, nitro, sulfonyl.

4. A sulfonamide derivative selected from the following group:
5-{[(3-methoxybenzoyl)amino]methyl}-2-[(4-{3-[(trifluoromethyl)sulfonlyl]anilino} piperidin-1-yl)sulfonyl]thiophene-3-carboxylic acid;
5-{[(3-methoxybenzoyl)amino]methyl}-2-{[4-(octylamino)piperidin-lyl] sulfonyl}thiophene-3-carboxylic acid;
N-(2-hydroxyethyl)-5-{[(3-methoxybenzoyl)amino]methyl}-2-[(4-{3-[(trifluoromethyl)
sulfonyl]anilino}piperidin-1-yl)sulfonyl]thiophene-3-carboxamide;
N-({4-(hydrazinocarbonyl)-5-[(4-{3-[(trifluoromethyl)sulfonyl]anilino}piperidin-1-yl)sulfonyl]thien-2-yl}methyl)-3-methoxybenzamide;
5-{[(3-methoxybenzoyl)amino]methyl}-2-[(4-{3-[(trifluoromethyl)sulfonyl]anilino}
piperidin-1-yl)sulfonyl]thiophene-3-carboxamide;
N-[2-(dimethylamino)ethyl]-5-{[(3-methoxybenzoyl)amino]methyl}-2-[(4-{3[( trifluoromethyl)sulfonyl]anilino}piperidin-1-yl)sulfonyl]thiophene-3carboxamide;
N-({4-(hydroxymethyl)-5-[(4-{3-[(trifluoromethyl)sulfonyl]anilino}piperidin-lyl) sulfonyl]thien-2-yl}methyl)-3-methoxybenzamide.

5. A sulfonamide derivative according to any of the preceding claims, for use as a medicament.

6. Use of a sulfonamide derivative according to any of claims 1-4 for the preparation of a medicament for the treatment of a neuronal disorder selected from epilepsy, Alzheimer's disease, Huntington's disease, Parkinson's disease, retinal diseases, spinal cord injury, Multiple Sclerosis, head trauma and ischemia, an auto-immune disease selected from inflammatory bowel disease (IBD), rheumatoid arthritis, asthma, septic shock, transplant rejection, a cancer selected from breast-, colorectal-, pancreatic, ovarian, prostate, testicular, hepatic, kidney, lung cancer, a cardiovascular disease including stroke, arterosclerosis, myocardial infarction, myocordial reperfusion injury and an ischemic condition including heart, renal, kidney and brain reperfusion injuries, renal failure.

7. A pharmaceutical composition containing at least one sulfonamide derivative according to any of the claims 1 to 4 and a pharmaceutically acceptable carrier, diluent or excipient thereof.

8. Process for the preparation of a sulfonamide derivative according to any of claims 1 to 4, wherein a sulfonyl chloride V 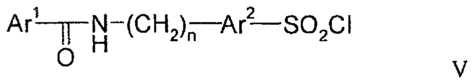 is reacted with a cyclic amide IX 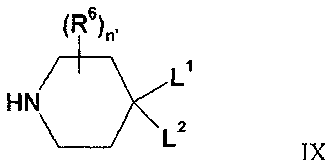 whereby (R⁶)ₙ, L¹ and L² are as above defined.

9. A process according to claim 8, wherein a sulfonyl chloride V is obtainable by
a) coupling an amine of formula II: 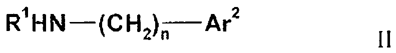 where Ar² and R¹ are as defined above, with an acyl chloride of formula III: 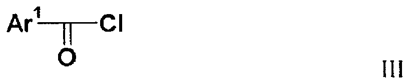 where Ar¹ is as defined above, to provide an amide of formula IV: 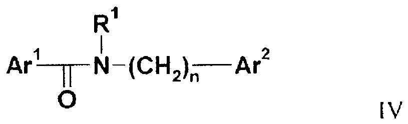
b) sulfonating the amide of formula IV to provide a sulfonyl chloride V

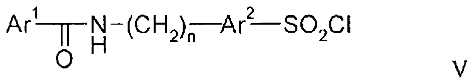

## Patentansprüche

1. Hydrophile Sulfonamidderivate gemäß Formel I 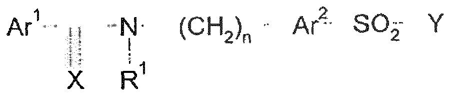 mit ihren geometrischen Isomeren, in einer optisch aktiven Form als Enantiomere, Diastereomere,
als auch in der Form von Racematen und den pharmazeutisch verträglichen Salzen davon, worin
Ar¹ Phenyl ist, das optional mit -OR substituiert ist, worin R C₁-C₆-Alkyl ist;
Ar² eine Thienylgruppe ist, die mindestens einen hydrophilen Substituenten trägt, worin der hydrophile Substituent -COOR³, -CONR³R^{3'}, OH, C₁-C₄-Alkyl, substituiert mit OH oder einer Amin-Gruppe, eine Hydrazido-Carbonyl-Gruppe, ein Sulfat, ein Sulfonat, ein Amin oder ein Ammoniumsalz ist;
X O oder S ist;
R¹ Wasserstoff oder eine C₁-C₆-Alkylgruppe ist;
n eine ganze Zahl von 1 bis 3 ist;
Y die allgemeine Formel 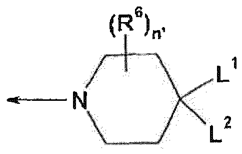 aufweist, wobei L¹ und L² unabhängig ausgewählt sind aus der Gruppe, bestehend aus H, -NR^{3'}R³, -NR³C(O)R³, -NR³C(O)NR^{3'}R³, - (SO)R³, -(SO₂)R³, -NSO₂R³, -SO₂NR^{3'}R³,
wobei R³, R^{3'} Substituenten sind, unabhängig ausgewählt aus der Gruppe, bestehend aus H, C₁-C₆-Alkyl, C₂-C₆-Alkenyl, Aryl, das Phenyl ist, Aryl-C₁-C₆-Alkyl, das Phenyl-C₁-C₆-Alkyl ist;
wobei die Arylgruppe optional substituiert ist mit Halogen, Hydroxy, Nitro, Sulfonyl;
R⁶ ausgewählt ist aus der Gruppe, bestehend aus Wasserstoff, C₁-C₆-Alkyl, C₁-C₆-Alkoxy, OH, Halogen, Nitro, Cyano, Sulfonyl, Oxo (=O), und
n' eine ganze Zahl von 0 bis 4 ist.

2. Sulfonamidderivat nach Anspruch 1, worin Ar¹ eine Phenylgruppe ist, optional substituiert mit -OR, worin R C₇-C₆-Alkyl ist, X O ist, R¹ Wasserstoff ist, n 1 ist, Ar² eine Thienylgruppe ist, die eine Gruppe trägt, ausgewählt aus -COOR³, -CONR³R^{3'}, OH, einem C₁-C₄-Alkyl, substituiert mit einer OH- oder Amino-Gruppe, einer Hydrazidocarbonyl-Gruppe.

3. Sulfonamidderivat nach Anspruch 2, worin Y 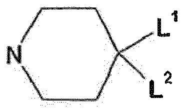 ist, wobei L² H ist, L¹ NHR³ ist, wobei R³ ein Substituent ist ausgewählt aus der Gruppe, bestehend aus C₁-C₆-Alkyl, einem Aryl, das Phenyl ist, Aryl-C₁-C₆-Alkyl, das Phenyl-C₁-C₆-Alkyl ist,
wobei die Arylgruppe optional substituiert ist mit Halogen, Hydroxy, Nitro, Sulfonyl.

4. Sulfonamidderivat, ausgewählt aus der folgenden Gruppe:
5-{[(3-Methoxybenzoyl)amino]methyl}-2-[(4-{3-[(trifluormethyl)sulfonyl]anilino}piperidin-1-yl)sulfonyl]thiophen-3-carbonsäure;
5-{[(3-Methoxybenzoyl)amino]methyl}-2-[4-octylamino)piperidin-1-yl)sulfonyl}thiophen-3-carbonsäure;
N-(2-Hydroxyethyl)-5-{[(3-methoxybenzoyl)amino]methyl}-2-[(4-{3-[(trifluormethyl)sulfonyl]anilino}piperidin-1-yl)sulfonyl]thiophen-3-carboxamid;
N-({4-(Hydrazinocarbonyl)-5-[(4-{3-[(trifluormethyl)sulfonyl]anilino}piperidin-1-yl)sulfonyl]thien-2-yl}methyl)-3-methoxybenzamid;
5-{[(3-Methoxybenzoyl)amino]methyl}-2-[(4-{3-[(trifluormethyl)sulfonyl]anilino}piperidin-1-yl)sulfonyl]thiophen-3-carboxamid;
N-[2-(Dimethylamino)ethyl)-5-{[(3-Methoxybenzoyl)amino]methyl}-2-[(4-{3-[(trifluormethyl)sulfonyl]anilino}piperidin-1-yl)sulfonyl]thiophen-3-carboxamid;
N-({4-(Hydroxymethyl)-5-[(4-{3-[(trifluormethyl)sulfonyl]anilino}piperidin-1-yl)sulfonyl]thien-2-yl}methyl)-3-methoxybenzamid.

5. Sulfonamidderivat nach einem der vorhergehenden Ansprüche zur Verwendung als ein Medikament.

6. Verwendung eines Sulfonamidderivats nach einem der Ansprüche 1-4 zur Herstellung eines Medikaments zur Behandlung einer neuronalen Störung, ausgewählt aus Epilepsie, Alzheimer-Krankheit, Huntington-Krankheit, Parkinsonkrankheit, Netzhauterkrankungen, Rückenmarksverletzung, Multiple Sklerose, Kopftrauma und Ischämie, einer Autoimmunerkrankung, ausgewählt aus entzündlicher Darmerkrankung (IBD), rheumatischer Arthritis, Asthma, septischer Schock, Transplantatabstoßung, einem Krebs, ausgewählt aus Brust-, Kolorektalkrebs, Pankreas-, Eierstock-, Prostata-, Hoden-, Leber-, Nieren-, Lungenkrebs, einer Herz-Kreislauf-Erkrankung, einschließlich Schlaganfall, Arteriosklerose, Herzinfarkt, Herzreperfusionsverletzung und ischämischem Zustand, einschließlich Herz, Niere, Nieren- und Hirnreperfusionsverletzungen, Nierenversagen.

7. Pharmazeutische Zusammensetzung, enthaltend mindestens ein Derivat nach einem der Ansprüche 1 bis 4 und einen pharmazeutisch verträglichen Träger, ein pharmazeutisch verträgliches Verdünnungsmittel oder einen pharmazeutisch verträglichen Exzipienten.

8. Verfahren zur Herstellung eines Sulfonamidderivats nach einem der Ansprüche 1 bis 4, worin ein Sulfonylchlorid V 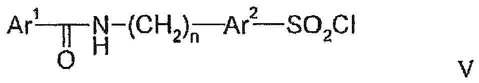 mit einem zyklischen Amid IX 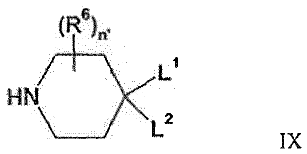 umgesetzt wird, wobei (R⁶)ₘ, L¹ und L² wie oben definiert sind.

9. Verfahren nach Anspruch 8, worin ein Sulfonylchlorid V erhältlich ist durch
a) Koppeln eines Amins der Formel II: 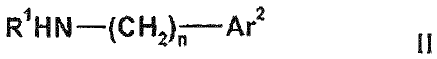 worin Ar² und R¹ wie oben definiert sind, mit einem Acylchlorid der Formel III: 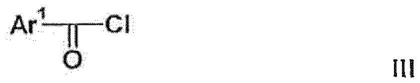 worin Ar¹ wie oben definiert ist, um ein Amid der Formel IV: 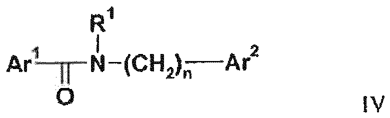 bereitzustellen,
b) Sulfonieren des Amids von Formel IV, um ein Sulfonylchlorid V 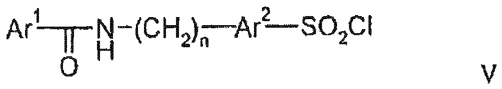 bereitzustellen.

## Revendications

1. Dérivés hydrophiles de type sulfonamide de formule I : 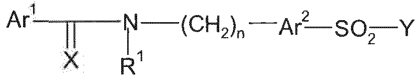 ainsi que leurs isomères géométriques, leurs formes optiquement actives, énantiomères et diastéréoisomères, leurs formes racémiques, et leurs sels pharmacologiquement admissibles,
dans laquelle formule :
- Ar¹ représente un groupe phényle, en option porteur d'un substituant symbolisé par -OR où R représente un groupe alkyle en C₁₋₆ ;
- Ar² représente un groupe thiényle, porteur d'au moins un substituant hydrophile, lequel substituant hydrophile est un groupe symbolisé par -COOR³ ou -CONR³R^{3'} un groupe hydroxyle, un groupe alkyle en C₁₋₄ porteur d'un substituant hydroxyle ou amino, un groupe hydrazido-carbonyle, un groupe de type sulfate ou sulfonate, ou un groupe de type amine ou sel d'ammonium ;
- X représente un atome d'oxygène ou de soufre ;
- R¹ représente un atome d'hydrogène ou un groupe alkyle en C₁₋₆ ;
- l'indice n est un nombre entier valant de 1 à 3 ;
- et Y représente un groupe de formule générale 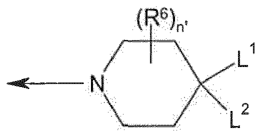 dans laquelle
- L¹ et L¹ représentent indépendamment des entités choisies dans l'ensemble formé par un atome d'hydrogène et les groupes symbolisés par -NR³'R³, -NR³'C(O)R³, -NR³'C(O)NR³'R³, -(SO)R³, -(SO₂)R³, -NSO₂R³ et -SO₂NR³'R³,
étant entendu que R³ et R³ représentent des substituants choisis indépendamment dans l'ensemble formé par un atome d'hydrogène et les groupes alkyle en C₁₋₆, alcényle en C₂₋₆, aryle signifiant phényle, et aryl-(alkyle en C₁₋₆) signifiant phényl-(alkyle en C₁₋₆), ledit groupe aryle portant, en option, un ou des substituant(s) halogéno, hydroxy, nitro et/ou sulfonyle,
- R⁶ représente une entité choisie dans l'ensemble formé par les atomes d'hydrogène et d'halogène, les groupes alkyle en C₁₋₆, alcoxy en C₁₋₆, hydroxyle, nitro, cyano et sulfonyle, et le substituant oxo (=O),
- et l'indice n' est un nombre entier valant de 0 à 4.

2. Dérivé de type sulfonamide, conforme à la revendication 1, dans lequel Ar¹ représente un groupe phényle, en option porteur d'un substituant symbolisé par -OR où R représente un groupe alkyle en C₁₋₆, X représente un atome d'oxygène, R¹ représente un atome d'hydrogène, l'indice n vaut 1, et Ar² représente un groupe thiényle, porteur d'un seul substituant choisi parmi un groupe symbolisé par -COOR³ ou -CONR³R³', un groupe hydroxyle, un groupe alkyle en C₁₋₄ porteur d'un substituant hydroxyle ou amino, et un groupe hydrazido-carbonyle.

3. Dérivé de type sulfonamide, conforme à la revendication 2, dans lequel Y représente un groupe de formule 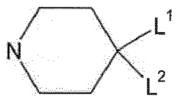 dans laquelle L² représente un atome d'hydrogène et L¹ représente un groupe symbolisé par -NHR³ où R³ représente un substituant choisi dans l'ensemble formé par les groupes alkyle en C₁₋₆, aryle signifiant phényle, et aryl-(alkyle en C₁₋₆) signifiant phényl-(alkyle en C₁₋₆), ledit groupe aryle portant, en option, un ou des substituant(s) halogéno, hydroxy, nitro et/ou sulfonyle.

4. Dérivé de type sulfonamide, choisi dans l'ensemble constitué par les suivants :
- acide 5-{[(3-méthoxy-benzoyl)-amino]-méthyl}-2-[(4-{3-[(trifluorométhyl)-sulfonyl]-anilino}-pipéridin-1-yl)-sulfonyl]-thiophène-3-carboxylique ;
- acide 5-{[(3-méthoxy-benzoyl)-amino]-méthyl}-2-{[4-(octyl-amino)-pipéridin-1-yl]-sulfonyl}-thiophéne-3-carboxylique ;
- N-(2-hydroxy-éthyl)-5-{[(3-méthoxy-benzoyl)-amino]-méthyl}-2-[(4-{3-[(trifluoro-méthyl)-sulfonyl]-anilino}-pipéridin-1-yl)-sulfonyl]-thiophéne-3-carboxamide ;
- N-({4-(hydrazino-carbonyl)-5-[(4-{3-[(trifluoro-méthyl)-sulfonyl]-anilino}-pipéridin-1-yl)-sulfonyl]-thién-2-yl}-méthyl)-3-méthoxy-benzamide ;
- 5-{[(3-méthoxy-benzoyl)-amino]-méthyl}-2-[(4-{3-[(trifluoro-méthyl)-sulfonyl]-anilino}-pipéridin-1-yl)-sulfonyl]-thiophène-3-carboxamide ;
- N-[2-(diméthyl-amino)-éthyl]-5-{[(3-méthoxy-benzoyl)-amino]-méthyl}-2-[(4-{3-[(trifluoro-méthyl)-sulfonyl]-anilino}-pipéridin-1-yl)-sulfonyl]-thiophéne-3-carboxamide ;
- N-({4-(hydroxy-méthyl)-5-[(4-{3-[(trifluoro-méthyl)-sulfonyl]-anilino}-pipéridin-1-yl)-sulfonyl]-thién-2-yl}-méthyl)-3-méthoxy-benzamide.

5. Dérivé de type sulfonamide, conforme à l'une des revendications précédentes, pour utilisation en tant que médicament.

6. Utilisation d'un dérivé de type sulfonamide, conforme à l'une des revendications 1 à 4, en vue de la préparation d'un médicament destiné au traitement d'un trouble neuronal choisi parmi les épilepsie, maladie d'Alzheimer, chorée de Huntington, maladie de Parkinson, troubles rétiniens, lésion de la moelle épinière, sclérose en plaques, traumatisme crânien et ischémie, d'une maladie auto-immune choisie parmi les affections intestinales inflammatoires (AII), polyarthrite rhumatoïde, asthme, choc septique et rejet d'organe transplanté, d'un cancer choisi parmi le cancer colorectal et les cancers du sein, du pancréas, des ovaires, de la prostate, des testicules, du foie, du rein et du poumon, d'une maladie cardio-vasculaire, y compris les attaques, artériosclérose, infarctus du myocarde et lésion de reperfusion du myocarde, d'un état ischémique incluant des lésions de reperfusion cardiaques, rénales ou cérébrales, ou d'une insuffisance rénale.

7. Composition pharmaceutique contenant au moins un dérivé de type sulfonamide, conforme à l'une des revendications 1 à 4, et un véhicule, diluant ou excipient pharmacologiquement admissible pour celui-ci.

8. Procédé de préparation d'un dérivé de type sulfonamide conforme à l'une des revendications 1 à 4, dans lequel on fait réagir un chlorure de sulfonyle de formule V : 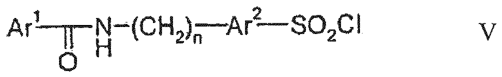 avec une amine cyclique de formule IX : 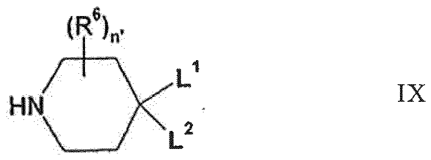 dans laquelle (R⁶)_{n'}, L¹ et L² ont les significations indiquées plus haut.

9. Procédé conforme à la revendication 8, pour lequel le chlorure de sulfonyle de formule V peut être obtenu par les opérations suivantes :
a) couplage d'une amine de formule II : 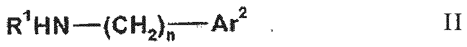 dans laquelle Ar² et R¹ ont les significations indiquées plus haut, avec un chlorure d'acyle de formule III : 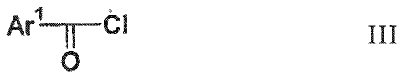 dans laquelle Ar¹ a la signification indiquée plus haut, réalisé de manière à obtenir un amide de formule IV : 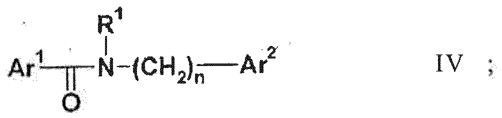
b) et sulfonation de cet amide de formule IV, réalisée de manière à obtenir un chlorure de sulfonyle de formule V :
